# FASCICULE DE BREVET EUROPEEN

(11) **EP 1 504 266 B1**
(45) Date de publication et mention de la délivrance du brevet: **07.08.2013**
(21) Numéro de dépôt: 03749938.1
(22) Date de dépôt: 09.05.2003
(51) Int. Cl.: G01N 33/576, G01N 33/569, C07K 16/10, C07K 16/42, C07K 14/005

(54) **PROCEDE DE DETECTION SIMULTANEE D' UN ANTIGENE ET D'UN ANTICORPS D'UN MICROORGANISME INFECTIEUX**
VERFAHREN ZUM GLEICHZEITIGEN NACHWEIS EINES ANTIGENS UND EINES ANTIKÖRPERS EINES INFEKTIÖSEN MIKROORGANISMUS
METHOD FOR SIMULTANEOUSLY DETECTING AN ANTIGEN AND AN ANTIBODY OF AN INFECTIOUS MICROORGANISM

(30) Priorité: 10.05.2002 FR 0205808
(43) Date de publication de la demande: 09.02.2005
(73) Titulaire: Bio-Rad Innovations, 92430 Marnes-La-Coquette (FR)
(72) Inventeur: RIEUNIER, François, F-78390 Bois d'Arcy (FR); FEYSSAGUET, Muriel, F-92210 Saint Cloud (FR); HENRIOT, Stéphanie, F-92150 Suresnes (FR); LAMBERT, Nadine, F-78400 Chatou (FR)
(74) Mandataire: Colombet, Alain André
(86) Numéro de dépôt international: PCT/FR2003/001429
(87) Numéro de publication internationale: WO 2003/095968

(56) Documents cités:
- EP-A- 0 363 025
- EP-A- 0 388 232
- EP-A- 0 442 394
- EP-A- 0 451 891
- WO-A-01/04149
- WO-A-92/22571
- WO-A-93/03376
- WO-A-95/23973
- WO-A-95/33206
- WO-A-98/40744
- WO-A-99/45395
- GB-A- 2 313 666
- KUO G ET AL: "AN ASSAY FOR CIRCULATING ANTIBODIES TO A MAJOR ETIOLOGIC VIRUS OF HUMAN NON-A, NON-B HEPATITIS" SCIENCE, AMERICAN ASSOCIATION FOR THE ADVANCEMENT OF SCIENCE,, US, vol. 244, no. 4902, 21 avril 1989 (1989-04-21), pages 362-364, XP000051651 ISSN: 0036-8075
- DATABASE EMBL [en ligne] Core Protein (genome polyprotein), Database accession no. q68685 XP002237193 & STUYVER L ET AL: "HEPATITIS C VIRUS GENOTYPING BY MEANS OF 5'-UR/CORE LINE PROBE ASSAYS AND MOLECULAR ANALYSIS OF UNTYPEABLE SAMPLES" VIRUS RESEARCH, AMSTERDAM, NL, vol. 38, 1995, pages 137-157, XP002057867 ISSN: 0168-1702
- KALININA O. ET AL: "A natural intergenotypic recombinant of hepatitis C virus identified in St. Petersburg" JOURNAL OF VIROLOGY, vol. 76, no. 8, avril 2002 (2002-04), pages 4034-4043, XP002237192 & DATABASE EMBL [en ligne] Core protein (genome polyprotein), Database accession no. q8qp59

### SEQUENCE LISTING

<110> BIORAD PASTEUR
<120> Procédé de détection simultanée d'un antigène et d'un anticorps d
   microorganisme infectieux
<130> BET 03P0457
<150> FR 0205808 °
   <151> 2002-05-10
<160> 33
<170> PatentIn version 3.1
<210> 1
   <211> 75
   <212> PRT
   <213> Hepatitis C virus
<400> 1
   Met Ser Thr Asn Pro Lys Pro Gln Arg Lys Thr Lys Arg Asn Thr Asn
   1               5                   10                  15
   Arg Arg Pro Gln Asp Val Lys Phe Pro Gly Gly Gly Gln Ile Val Gly
               20                  25                  30
   Gly Val Tyr Leu Leu Pro Arg Arg Gly Pro Arg Leu Gly Val Arg Ala
           35                  40                  45
   Thr Arg Lys Thr Ser Glu Arg Ser Gln Pro Arg Gly Arg Arg Gln Pro
       50                  55                  60
   Ile Pro Lys Ala Arg Arg Pro Glu Gly Arg Ser
   65                  70                  75
<210> 2
   <211> 63
   <212> PRT
   <213> Hepatitis C virus
<400> 2
   Lys Pro Gln Arg Lys Thr Lys Arg Asn Thr Asn Arg Arg Pro Gln Asp
   1               5                   10                  15
   Val Lys Phe Pro Gly Gly Gly Gln Ile Val Gly Gly Val Tyr Leu Leu
               20                  25                  30
   Pro Arg Arg Gly Pro Arg Leu Gly Val Arg Ala Thr Arg Lys Thr Ser
           35                  40                  45
   Glu Arg Ser Gln Pro Arg Gly Arg Arg Gln Pro Ile Pro Lys Ala
       50                  55                  60
<210> 3
   <211> 53
   <212> PRT
   <213> Hepatitis C virus
<400> 3
   Met Ser Thr Asn Pro Lys Pro Gln Arg Lys Thr Lys Arg Asn Thr Asn
   1               5                   10                  15
   Arg Arg Pro Gln Asp Val Lys Phe Pro Gly Gly Gly Gln Ile Val Gly
               20                  25                  30
   Gly Val Tyr Leu Leu Pro Arg Arg Gly Pro Arg Leu Gly Val Arg Ala
           35                  40                  45
   Thr Arg Lys Thr Ser
       50
<210> 4
   <211> 75
   <212> PRT
   <213> Hepatitis C virus
<400> 4
   Met Ser Thr Asn Pro Lys Pro Gln Arg Lys Thr Lys Arg Asn Thr Asn
   1               5                   10                  15
   Arg Arg Pro Gln Asp Val Lys Phe Pro Gly Gly Gly Gln Ile Val Gly
               20                  25                  30
   Gly Gly Tyr Leu Leu Pro Arg Arg Gly Pro Arg Gly Gly Val Gly Ala
           35                  40                  45
   Thr Arg Lys Thr Ser Glu Arg Ser Gln Pro Arg Gly Arg Arg Gln Pro
       50                  55                  60
   Ile Pro Lys Ala Arg Arg Pro Glu Gly Arg Ser
   65                  70                  75
<210> 5
   <211> 75
   <212> PRT
   <213> Hepatitis C virus
<400> 5
   Met Ser Thr Asn Pro Lys Pro Gln Arg Lys Thr Lys Arg Asn Thr Asn
   1 5 10 15
   Arg Arg Pro Gln Asp Val Lys Phe Pro Gly Gly Gly Gln Ile Gly Gly
   20 25 30
   Gly Val Tyr Leu Leu Pro Arg Arg Gly Pro Arg Gly Gly Val Gly Ala
   35 40 45
   Thr Arg Lys Thr Ser Glu Arg Ser Gln Pro Arg Gly Arg Arg Gln Pro
   50 55 60
   Ile Pro Lys Ala Arg Arg Pro Glu Gly Arg Ser
   65 70 75
<210> 6
   <211> 75
   <212> PRT
   <213> Hepatitis C virus
<400> 6
   Met Ser Thr Asn Pro Lys Pro Gln Arg Lys Thr Lys Arg Asn Thr Asn
   1 5 10 15
   Arg Arg Pro Gln Asp Val Lys Phe Pro Gly Gly Gly Gln Ile Val Gly
   20 25 30
   Gly Val Tyr Gly Leu Pro Arg Arg Gly Pro Arg Gly Gly Val Gly Ala
   35 40 45
   Thr Arg Lys Thr Ser Glu Arg Ser Gln Pro Arg Gly Arg Arg Gln Pro
   50 55 60
   Ile Pro Lys Ala Arg Arg Pro Glu Gly Arg Ser
   65 70 75
<210> 7
   <211> 63
   <212> PRT
   <213> Hepatitis C virus
<400> 7
   Lys Pro Gln Arg Lys Thr Lys Arg Asn Thr Asn Arg Arg Pro Gln Asp
   1 5 10 15
   Val Lys Phe Pro Gly Gly Gly Gln Ile Val Gly Gly Gly Tyr Leu Leu
   20 25 30
   Pro Arg Arg Gly Pro Arg Gly Gly Val Gly Ala Thr Arg Lys Thr Ser
   35 40 45
   Glu Arg Ser Gln Pro Arg Gly Arg Arg Gln Pro Ile Pro Lys Ala
   50 55 60
<210> 8
   <211> 63
   <212> PRT
   <213> Hepatitis C virus
<400> 8
   Lys Pro Gln Arg Lys Thr Lys Arg Asn Thr Asn Arg Arg Pro Gln Asp
   1 5 10 15
   Val Lys Phe Pro Gly Gly Gly Gln Ile Gly Gly Gly Val Tyr Leu Leu
   20 25 30
   Pro Arg Arg Gly Pro Arg Gly Gly Val Gly Ala Thr Arg Lys Thr Ser
   35 40 45
   Glu Arg Ser Gln Pro Arg Gly Arg Arg Gln Pro Ile Pro Lys Ala
   50 55 60
<210> 9
   <211> 70
   <212> PRT
   <213> Hepatitis C virus
<400> 9
   Lys Pro Gln Arg Lys Thr Lys Arg Asn Thr Asn Arg Arg Pro Gln Asp
   1 5 10 15
   Val Lys Phe Pro Gly Gly Gly Gln Ile Val Gly Gly Val Tyr Gly Leu
   20 25 30
   Pro Arg Arg Gly Pro Arg Gly Gly Val Gly Ala Thr Arg Lys Thr Ser
   35 40 45
   Glu Arg Ser Gln Pro Arg Gly Arg Arg Gln Pro Ile Pro Lys Ala Arg
   50 55 60
   Arg Pro Glu Gly Arg Ser
   65 70
<210> 10
   <211> 53
   <212> PRT
   <213> Hepatitis C virus
<400> 10
   Met Ser Thr Asn Pro Lys Pro Gln Arg Lys Thr Lys Arg Asn Thr Asn
   1               5                   10                  15
   Arg Arg Pro Gln Asp Val Lys Phe Pro Gly Gly Gly Gln Ile Val Gly
               20                  25                  30
   Gly Gly Tyr Leu Leu Pro Arg Arg Gly Pro Arg Gly Gly Val Gly Ala
           35                  40                  45
   Thr Arg Lys Thr Ser
       50
<210> 11
   <211> 53
   <212> PRT
   <213> Hepatitis C virus
<400> 11
   Met Ser Thr Asn Pro Lys Pro Gln Arg Lys Thr Lys Arg Asn Thr Asn
   1               5                   10                  15
   Arg Arg Pro Gln Asp Val Lys Phe Pro Gly Gly Gly Gln Ile Gly Gly
               20                  25                  30
   Gly Val Tyr Leu Leu Pro Arg Arg Gly Pro Arg Gly Gly Val Gly Ala
           35                  40                  45
   Thr Arg Lys Thr Ser
       50
<210> 12
   <211> 53
   <212> PRT
   <213> Hepatitis C virus
<400> 12
   Met Ser Thr Asn Pro Lys Pro Gln Arg Lys Thr Lys Arg Asn Thr Asn
   1               5                   10                  15
   Arg Arg Pro Gln Asp Val Lys Phe Pro Gly Gly Gly Gln Ile Val Gly
               20                  25                  30
   Gly Val Tyr Gly Leu Pro Arg Arg Gly Pro Arg Gly Gly Val Gly Ala
   35 40 45
   Thr Arg Lys Thr Ser
   50
<210> 13
   <211> 73
   <212> PRT
   <213> Hepatitis C virus
<400> 13
   Met Ser Thr Asn Pro Lys Pro Gln Arg Lys Thr Lys Arg Asn Thr Asn
   1 5 10 15
   Arg Arg Pro Gln Asp Val Lys Phe Pro Gly Gly Gly Gln Ile Val Gly
   20 25 30
   Gly Gly Tyr Leu Leu Pro Arg Arg Gly Pro Arg Leu Arg Ala Thr Arg
   35 40 45
   Lys Thr Ser Glu Arg Ser Gln Pro Arg Gly Arg Arg Gln Pro Ile Pro
   50 55 60
   Lys Ala Arg Arg Pro Glu Gly Arg Ser
   65 70
<210> 14
   <211> 75
   <212> PRT
   <213> Hepatitis C virus
<220>
   <221> MOD_RES
   <222> (45) .. (45)
   <223> bAla
<400> 14
   Met Ser Thr Asn Pro Lys Pro Gln Arg Lys Thr Lys Arg Asn Thr Asn
   1 5 10 15
   Arg Arg Pro Gln Asp Val Lys Phe Pro Gly Gly Gly Gln Ile Val Gly
   20 25 30
   Gly Gly Tyr Leu Leu Pro Arg Arg Gly Pro Arg Leu Ala Val Arg Ala
   35 40 45
   Thr Arg Lys Thr Ser Glu Arg Ser Gln Pro Arg Gly Arg Arg Gln Pro
   50 55 60
   Ile Pro Lys Ala Arg Arg Pro Glu Gly Arg Ser
   65 70 75
<210> 15
   <211> 76
   <212> PRT
   <213> Hepatitis C virus
<400> 15
   Met Ser Thr Asn Pro Lys Pro Gln Arg Lys Thr Lys Arg Asn Thr Asn
   1 5 10 15
   Arg Arg Pro Gln Asp Val Lys Phe Pro Gly Gly Gly Gln Ile Val Gly
   20 25 30
   Gly Gly Tyr Leu Leu Pro Arg Arg Gly Pro Arg Leu Gly Gly Gly Arg
   35 40 45
   Ala Thr Arg Lys Thr Ser Glu Arg Ser Gln Pro Arg Gly Arg Arg Gln
   50 55 60
   Pro Ile Pro Lys Ala Arg Arg Pro Glu Gly Arg Ser
   65 70 75
<210> 16
   <211> 75
   <212> PRT
   <213> Hepatitis C virus
<220>
   <221> MOD_RES
   <222> (29) .. (29)
   <223> Nle
<400> 16
   Met Ser Thr Asn Pro Lys Pro Gln Arg Lys Thr Lys Arg Asn Thr Asn
   1 5 10 15
   Arg Arg Pro Gln Asp Val Lys Phe Pro Gly Gly Gly Leu Gly Val Gly
   20 25 30
   Gly Val Tyr Leu Leu Pro Arg Arg Gly Pro Arg Gly Gly Val Gly Ala
   35 40 45
   Thr Arg Lys Thr Ser Glu Arg Ser Gln Pro Arg Gly Arg Arg Gln Pro
   50 55 60
   Ile Pro Lys Ala Arg Arg Pro Glu Gly Arg Ser
   65 70 75
<210> 17
   <211> 75
   <212> PRT
   <213> Hepatitis C virus
<400> 17
   Met Ser Thr Asn Pro Lys Pro Gln Arg Lys Thr Lys Arg Asn Thr Asn
   1 5 10 15
   Arg Arg Pro Gln Asp Val Lys Phe Pro Gly Gly Gly Gln Ile Val Gly
   20 25 30
   Gly Gly Phe Leu Leu Pro Arg Arg Gly Pro Arg Gly Gly Val Gly Ala
   35 40 45
   Thr Arg Lys Thr Ser Glu Arg Ser Gln Pro Arg Gly Arg Arg Gln Pro
   50 55 60
   Ile Pro Lys Ala Arg Arg Pro Glu Gly Arg Ser
   65 70 75
<210> 18
   <211> 75
   <212> PRT
   <213> Hepatitis C virus
<220>
   <221> MISC_FEATURE
   <222> (35) .. (35)
   <223> homo-serine
<400> 18
   Met Ser Thr Asn Pro Lys Pro Gln Arg Lys Thr Lys Arg Asn Thr Asn
   1 5 10 15
   Arg Arg Pro Gln Asp Val Lys Phe Pro Gly Gly Gly Gln Ile Val Gly
   20 25 30
   Gly Gly Ser Leu Leu Pro Arg Arg Gly Pro Arg Gly Gly Val Gly Ala
   35 40 45
   Thr Arg Lys Thr Ser Glu Arg Ser Gln Pro Arg Gly Arg Arg Gln Pro
   50 55 60
   Ile Pro Lys Ala Arg Arg Pro Glu Gly Arg Ser
   65 70 75
<210> 19
   <211> 4
   <212> PRT
   <213> Hepatitis C virus
<400> 19
   Leu Gly Val Arg
   1
<210> 20
   <211> 7
   <212> PRT
   <213> Hepatitis C virus
<400> 20
   Ile Val Gly Gly Val Tyr Leu
   1 5
<210> 21
   <211> 6
   <212> PRT
   <213> Hepatitis C virus
<400> 21
   Gln Ile Val Gly Gly Val
   1 5
<210> 22
   <211> 58
   <212> PRT
   <213> Human immunodeficiency virus type 1
<400> 22
   Phe Arg Gly Tyr Val Gly Arg Phe Tyr Lys Thr Leu Arg Ala Glu Gln
   1 5 10 15
   Ala Gly Gln Gly Val Lys Asn Phe Met Thr Glu Thr Leu Leu Val Gln
   20 25 30
   Asn Ala Asn Pro Asp Cys Lys Thr Ile Leu Lys Ala Leu Gly Pro Ala
   35 40 45
   Ala Thr Leu Glu Glu Met Met Thr Ala Cys
   50 55
<210> 23
   <211> 58
   <212> PRT
   <213> Human immunodeficiency virus type 1
<400> 23
   Phe Arg Asp Tyr Val Asp Arg Phe Tyr Lys Thr Leu Arg Ala Glu Gln
   1 5 10 15
   Ala Ser Gln Glu Val Lys Asn Trp Met Thr Glu Thr Leu Leu Val Gln
   20 25 30
   Asn Ala Asn Pro Asp Cys Lys Thr Ile Leu Lys Ala Leu Gly Pro Ala
   35 40 45
   Ala Thr Leu Glu Glu Met Met Thr Ala Cys
   50 55
<210> 24
   <211> 15
   <212> PRT
   <213> Human immunodeficiency virus type 1
<400> 24
   Gln Ala Ser Gln Glu Val Lys Asn Trp Met Thr Glu Thr Leu Leu
   1 5 10 15
<210> 25
   <211> 15
   <212> PRT
   <213> Human immunodeficiency virus type 2
<400> 25
   Gln Thr Asp Pro Ala Val Lys Asn Trp Met Thr Gln Thr Leu Leu
   1 5 10 15
<210> 26
   <211> 12
   <212> PRT
   <213> Human immunodeficiency virus type 1
<400> 26
   Leu Gly Leu Trp Gly Cys Ser Gly Lys Leu Ile Cys
   1 5 10
<210> 27
   <211> 12
   <212> PRT
   <213> Human immunodeficiency virus type 1
<400> 27
   Leu Gly Ile Trp Gly Cys Ser Gly Lys Leu Ile Cys
   1 5 10
<210> 28
   <211> 12
   <212> PRT
   <213> Human immunodeficiency virus type 1
<400> 28
   Leu Gly Leu Trp Gly Cys Ser Gly Lys His Ile Cys
   1 5 10
<210> 29
   <211> 12
   <212> PRT
   <213> Human immunodeficiency virus type 1
<400> 29
   Leu Gly Met Trp Gly Cys Ser Gly Lys His Ile Cys
   1 5 10
<210> 30
   <211> 26
   <212> PRT
   <213> Human immunodeficiency virus type 1
<400> 30
   Arg Ile Leu Ala Val Glu Arg Tyr Leu Lys Asp Gln Gln Leu Leu Gly
   1 5 10 15
   Ile Trp Gly Cys Ser Gly Lys Leu Ile Cys
   20 25
<210> 31
   <211> 34
   <212> PRT
   <213> Human immunodeficiency virus type 1
<400> 31
   Arg Ile Leu Ala Val Glu Arg Tyr Leu Lys Asp Gln Gln Leu Leu Gly
   1 5 10 15
   Ile Trp Gly Ser Gly Lys Leu Ile Cys Thr Thr Ala Val Pro Trp Asn
   20 25 30
   Ala Ser
<210> 32
   <211> 12
   <212> PRT
   <213> Human immunodeficiency virus type 2
<400> 32
   Leu Asn Ser Trp Gly Cys Ala Phe Arg Gln Val Cys
   1               5                   10
<210> 33
   <211> 36
   <212> PRT
   <213> Human immunodeficiency virus type 2
<400> 33
   Arg Val Thr Ala Ile Glu Lys Tyr Leu Gln Asp Gln Ala Arg Leu Asn
   1               5                   10                  15
   Ser Trp Gly Cys Ala Phe Arg Gln Val Cys His Thr Thr Val Pro Trp
               20                  25                  30
   Val Asn Asp Ser
           35

## Description

L'invention concerne la détection *in vitro* d'une infection par un microorganisme infectieux, notamment viral, et en particulier la détection *in vitro* d'une infection par un virus de l'hépatite C (VHC). L'invention concerne plus précisément aussi un procédé de détection simultanée d'un antigène d'un microorganisme infectieux, notamment viral, et d'anticorps dirigés contre ce même microorganisme infectieux ainsi que les réactifs et trousses le mettant en oeuvre. Plus particulièrement, elle concerne un procédé de détection simultanée d'antigène VHC et d'anticorps anti-VHC, et les réactifs et trousses le mettant en oeuvre.

L'infection par le virus de l'hépatite C, une hépatite initialement appelée hépatite Non-A, Non-B, est un problème de santé préoccupant et reconnu depuis longtemps, en particulier en transfusion sanguine.

La demande de brevet EP 318 216 publiée le 31 Mai 1989 décrit le clonage de fragments de l'ADNc d'un virus responsable de l'hépatite C chez l'homme, dénommé HCV (en anglais) ou VHC (en français). Elle décrit aussi la séquence de cinq gènes codant les protéines non-structurales (NS1 à NS5) du virus (environ 78 % du génome total du VHC), l'antigène C100-3 (qui contient 363 acides aminés de la région NS3-NS4 et fusionné à la superoxyde dismutase) ainsi qu'un procédé de détection d'anticorps anti-VHC à l'aide de l'antigène C100-3. Ce procédé de détection d'anticorps anti-VHC, dit de première génération, a permis, entre autres, d'établir que le VHC est une cause majeure d'hépatite Non-A, Non-B, maintenant appelée hépatite C, dans le monde. Cependant, ce procédé ne permet pas de détecter plus de 70 à 80 % des sérums infectés par le virus. Ce manque de sensibilité ne permet pas non plus une détection précoce des infections.

Okamoto et al. (1990a) et la demande de brevet EP 388 232 publiée le 19 septembre 1990 décrivent la séquence terminale 5' du génome du virus VHC, c'est à dire la séquence des gènes codant les protéines structurales (capside, matrice, enveloppe) du virus responsable de l'hépatite C.

Okamoto et al. (1990b) publient l'utilisation de la séquence d'acides aminés 39-74 de la capside du VHC comme cible de détection ELISA d'anticorps anti-VHC.

L'article Hosein et al. (1991), décrit un immunoessai de détection d'anticorps anti-VHC fondé sur l'emploi d'antigènes peptidiques synthétiques structuraux (capside : dans la région AA 1-120) et non-structuraux (NS3-NS4 : dans la région AA 1200-1800). Il démontre l'intérêt des peptides synthétiques en détection d'anticorps anti-VHC et de la combinaison des antigènes structuraux et non-structuraux: elle augmente la sensibilité, et donc la précocité, de la détection. L'essai décrit ici permet une détection d'anticorps plus précoce de 4 à 10 semaines. L'article montre aussi qu'il n'existe pas d'épitope immunodominant majeur, comme on en connaît, par exemple, chez les virus du SIDA.

Nasoff et al. (1991) constatent que la plupart des épitopes immunoréactifs dominants de la capside sont localisés dans la région N-terminale (AA 1-40) et que les anticorps dirigés contre ces épitopes apparaissent précocément après l'infection.

Les essais de détection d'anticorps anti-VHC dits de deuxième génération (c'est à dire fondés sur l'utilisation simultanée d'antigènes de capture non-structuraux et structuraux) constituent un progrès significatif vis à vis des essais de première génération. Cependant, ils manquent encore de sensibilité : ils détectent en effet au mieux 95 à 98 % des sérums prélevés chez des patients contaminés par le VHC. Par voie de conséquence, cette détection est encore trop peu précoce et laisse encore passer inaperçus des dons de sang infecté en transfusion sanguine. En effet, pour réduire les risques post-transfusionnels, il est nécessaire de détecter la présence du virus lui-même, avant l'apparition d'anticorps, et le plus tôt possible après la contamination. Cette période entre la contamination et la séroconversion (c'est à dire l'apparition d'anticorps) est appelée « fenêtre sérologique ».

Différentes équipes (Garson et al. (1990) ; Shieh et al. (1991)) ont proposé la détection de l'ARN viral par la PCR (Réaction en chaîne par la polymerase) pour résoudre le problème de sensibilité et de précocité évoqué ci-dessus. Cette méthode permet en effet une détection extrêmement sensible et précoce de l'infection par le VHC, c'est à dire seulement quelques jours après l'exposition au virus, soit 4 à 8 semaines avant l'élévation des anticorps antiviraux circulants. Elle constitue actuellement la méthode de référence pour la détection du virus dans les fluides biologiques.

Toutefois, la méthode PCR appliquée au VHC se heurte à diverses difficultés. D'une part, elle implique la réalisation de pré-étapes de d'extraction, purification de l'ARN, puis de transcription inverse de l'ARN en ADNc, et une partie du matériel viral est perdue au cours de ces étapes préalables. D'autre part, elle requiert un équipement d'amplification spécifique et onéreux. De plus, elle ne permet pas de traiter simultanément un grand nombre d'échantillons et donne souvent lieu à des contaminations.

Une autre approche, en vue de la détection précoce de l'infection par le VHC, a consisté en la détection de l'antigène viral (capside) circulant. Cet antigène apparaît également plusieurs semaines avant l'apparition des anticorps anti-VHC sériques. Takahashi et al., (1992) décrivent une technique ELISA détectant l'antigène de capside à l'aide d'un couple d'anticorps.

Cependant, la détection de cet antigène est difficile à mettre au point en raison, en grande partie, du faible taux d'antigène détectable dans le sang et de la qualité des réactifs immunologiques disponibles.

Hajime Tokita et al. (2000) décrivent un immunoessai de type sandwich à l'aide d'un couple d'anticorps monoclonaux (5F11 et 5E3), connu sur le marché sous le nom « Immucheck F HCV Ag Core Kokusai », de détection très sensible. Les auteurs de cet article soulignent qu'une mutation Thr49Pro dans la protéine de capside réduit la sensibilité de l'essai. Cherchant également à détecter l'antigène de capside à un stade précoce, Peterson et al. (2000) expose une technique de détection ELISA de l'antigène de capside du VHC à l'aide d'anticorps monoclonaux anti-capside, sans prétraitement de l'échantillon. L'article montre, grâce à la comparaison de trois essais indépendants (détection de l'ARN du VHC par la PCR, des anticorps anti-VHC et de l'antigène de capside par ELISA), qu'ainsi l'antigène de capside circulant du VHC peut être détecté utilement dans des poches de sang prélevé durant la phase séronégative précoce de l'infection (soit environ 1 jour après la détection de l'ARN).

La précocité de la détection de l'infection par le virus de l'hépatite C associée à la possibilité de détecter les réponses anticorps postérieures à la séroconversion sur toute la durée de l'infection reste un objectif d'actualité, tout particulièrement en transfusion.

Dans l'optique de disposer d'une méthode simple, sensible, spécifique, reproductible, bon marché et de mise en oeuvre facile, automatisable - en vue du dépistage de masse - pour détecter, en premier lieu, l'antigène VHC pendant la période de la fenêtre sérologique, puis suivre l'évolution sérologique du patient après la séroconversion, combiner une détection des anticorps anti-VHC et une détection d'un antigène de VHC est tout à fait souhaitable.

Cependant, ceci pose un problème majeur, celui de l'interférence, sur le dosage de l'antigène de VHC, entre les anticorps anti-VHC présents dans le sérum et des anticorps anti-VHC marqués. Ainsi l'introduction sur une phase solide, en vue de la détection d'un anticorps donné, d'un antigène cible qui aurait les mêmes épitopes que ceux reconnus par le(s) anticorps marqué(s), utilisé(s) en vue de la détection simultanée en sandwich d'un antigène, entraînerait irrémédiablement une fixation d'anticorps marqué(s) sur la phase solide et donc une réponse faussement positive de l'essai.

Ceci est particulièrement vrai dans un système de détection simultanée, sur la même phase solide, d'anticorps anti-capside du VHC et d'antigène de capside du VHC. Ainsi le dépôt sur la phase solide, en vue de la détection d'anticorps anti capside du VHC, d'un antigène de capside qui a les mêmes épitopes que ceux reconnus par le(s) anticorps anti-capside VHC marqué(s) utilisé(s) en vue de la détection de l'antigène capsidique entraîne une fixation d'anticorps marqué(s) sur la phase solide et résulte en une réponse faussement positive de l'essai.

Pour contourner ce risque d'interférence, Chiron Corp. a réalisé des essais détectant l'antigène de capside et les seuls anticorps anti-NS3/NS4 de patients. Pour cela Chiron a mis en oeuvre d'une part un antigène NS3/4a fixé sur une phase solide, pour capturer les anticorps anti-VHC de l'échantillon testé, et d'autre part des anticorps monoclonaux (c11-3 et c11-7) anti-capside du VHC, également fixés. Les anticorps capturés étaient détectés par un antigène fusionné avec de la SOD (superoxyde dismutase) en présence d'un anticorps monoclonal marqué à la peroxydase, tandis que l'antigène capturé était détecté par un autre anticorps monoclonal marqué également à la peroxydase (VII European Congress of the International Society of Blood Transfusion - Paris, 15-18 juillet 2001).

Face au problème des interférences, la demande EP 1 020 727 (Advanced Life Science Institute) propose une méthode de mesure simultanée de l'antigène de capside VHC et des anticorps anti-capside VHC (un essai de type « Combo »), dans laquelle l'antigène est capturé et marqué par des anticorps dirigés contre des épitopes capsidiques différents des épitopes capsidiques servant simultanément à la capture et la révélation ou détection d'anticorps anti-capside. Un exemple représentatif est donné où, dans l'essai simultané de détection de l'antigène en sandwich et des anticorps en essai indirect, on utilise, pour détecter l'antigène, un premier anticorps (de capture), dirigé contre les épitopes de la séquence de l'acide aminé (AA) 100 à l'acide aminé 130 de la capside du VHC, et un deuxième anticorps (de détection), dirigé contre les épitopes de la séquence AA40-50, et, pour détecter les anticorps, l'antigène de capture utilisé contient pour sa part les séquences AA1-42 et AA66-80.

Cette méthode n'est cependant pas exempte d'inconvénients, en particulier parce qu'elle nécessite l'utilisation d'anticorps dirigés contre des épitopes relativement distants les uns des autres, et qui sont en fait des épitopes mineurs, peu immunogènes. Elle a l'inconvénient en plus, du fait de l'absence de la séquence AA43-65, de ne pas détecter les anticorps dirigés contre cette dernière séquence et donc de perdre en sensibilité.
De plus, elle réalise la capture de l'antigène capsidique et la capture des anticorps anti-capside par le biais de deux zones de la protéine de la capside clairement distinctes, i.e. non superposées (AA 1-42 et AA 66-80 pour détecter les anticorps et AA 100-130 pour détecter l'antigène) à la différence de la présente invention.

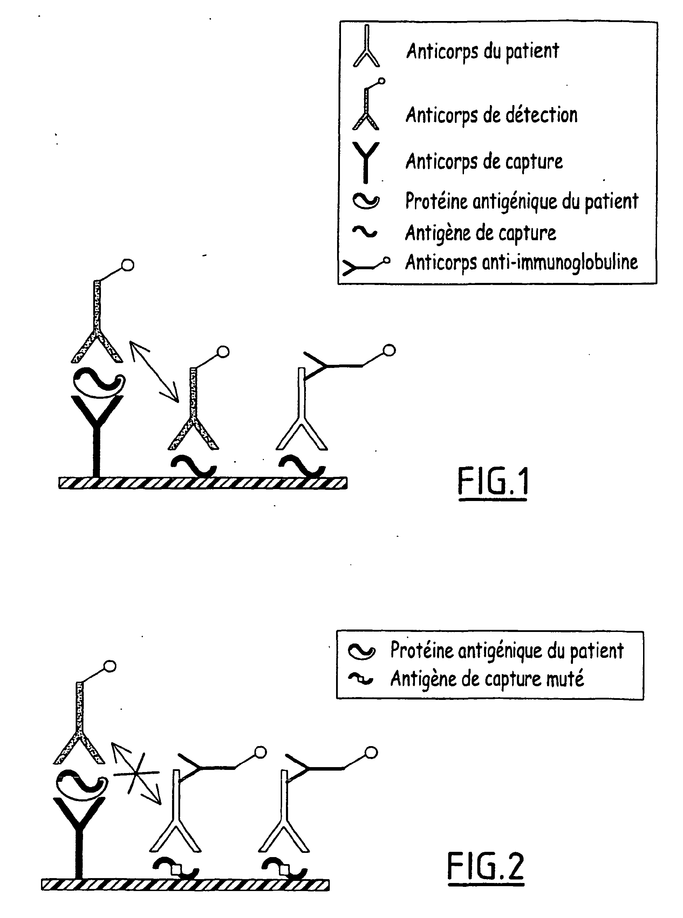

La demande de brevet WO 01/96875 A2 (CHIRON) décrit, entre autres, un essai de détection simultanée de la capside et des anticorps anti NS3 et NS4 (un « Combo incomplet », figure 2) faisant usage de N-laurylsarcosine comme détergent. Elle évoque, par contre, très sommairement, en figure 8 et page 33, un essai « Combo complet », i.e. un essai de détection simultanée de l'antigène de capside (en sandwich) et des anticorps anti-capside et anti-protéines non-structurales du VHC (en sandwich double antigène). On utilise, pour capturer l'antigène, deux anticorps, c11-3 et c11-7, réputés reconnaître une vaste portion N-terminale (AA 10-53) de la capside du VHC, et pour la détection un troisième anticorps, c11-14, réputé reconnaître une portion C-terminale (AA 120-130) de la capside du VHC. Pour détecter les anticorps, l'antigène de capture utilisé est un antigène de fusion à épitopes multiples (« MEFA 12 », voir tableau 2 de la demande WO 01/96875) qui contient, en fusion avec un fragment de superoxyde-dismutase (« SOD »), des antigènes NS3, NS4, NS5 et des séries de séquences capsidiques de plusieurs souches de VHC: AA 9-53, porteuse de la mutation R47L, AA 64-88 et AA 67-84. Les séquences AA 54-63 et AA 54-66 sont absentes de ces deux séries de séquence.

Toutefois, la mise en oeuvre réelle de l'essai « Combo complet » de la demande de brevet WO 01/96875 A2 n'est pas décrite. Il est donc impossible pour un homme du métier de déterminer clairement et sans ambiguïté si l'essai Combo de la figure 8 peut fonctionner, encore moins s'il peut satisfaire au problème posé, *i.e*. détecter aussi précocement que possible l'infection par un VHC. En tout état de cause, il est clair que, dans le meilleur des cas, le Combo de la demande de brevet WO 01/96875 A2 perdrait inévitablement la détection de tous les anticorps dirigés contre les séquences manquantes AA 54-63 et AA 54-66. Il s'ensuivrait un risque de perte de sensibilité.

La demande de brevet EP 1 251 353 A2 (Ortho-Clinical Diagnostics) décrit un essai « Combo complet » utilisant les mêmes anticorps pour la détection de la capside, sans toutefois préciser leur origine ni leur spécificité épitopique. En outre, elle précise que le détergent utilisé est un détergent de type BRIJ ou MYRJ, qui apparemment est préféré à la N-lauryl sarcosine de la trousse de détection de l'antigène capsidique commercialisée par Ortho Clinical Diagnostics (voir exemple 3 [0012]). La détection des anticorps anti-capside se fait à l'aide d'un antigène de capside modifié (par mutagenèse) : C22KSN∇47,48 (protéine de fusion avec la SOD comprenant la séquence capsidique AA 10-99 délétée des acides aminés 47 et 48) ou C22KSR47L (protéine de fusion avec la SOD comprenant la séquence capsidique AA 10-99, avec une leucine remplaçant une arginine en position 47).

La demande de brevet WO 03/002 749 A2 (Abbott) décrit de nombreux antigènes et essais de détection d'antigène capsidique du VHC. Le seul essai « Combo complet » qu'elle décrit - sous le nom de « Real Combo » (Figure 1, et page 59) fait usage, pour la détection des anticorps anti capside, d'un peptide biotinylé correspondant aux acides aminés 11-28 de la capside, immobilisé en phase solide. Pour la détection de la capside, elle met en oeuvre la combinaison d'anticorps d'Advanced Life Science Institute C11-14 (reconnaissant la séquence capsidique AA 45-50) en phase solide et C11-10 (reconnaissant la séquence capsidique AA 32-36) marqué à l'acridine. La demande WO 03/002 749 A2 réalise donc la capture de l'antigène capsidique et la capture des anticorps anti-capside par le biais de deux sites capsidiques clairement distincts, i.e. non superposés, (AA 11-28 pour détecter les anticorps et AA 45-50 pour détecter l'antigène) à la différence de la présente invention.

Les auteurs de la présente invention se sont alors attachés à mettre au point une méthode alternative pour résoudre le problème posé.

Il a été maintenant trouvé une méthode de détection simultanée d'un antigène de VHC et des anticorps d'un patient dirigés contre cet antigène qui évite ce problème d'interférence et qui atteint des niveaux de sensibilité et de précocité de détection qui se rapprochent de ceux de la PCR, tout en permettant de suivre l'évolution sérologique du patient après la séroconversion.

Les auteurs de l'invention résolvent ce problème en rendant artificiellement différents, par modification de structure, certains épitopes des antigènes cibles utilisés pour capturer les anticorps. Les épitopes ainsi modifiés sont alors détruits. Simultanément, les anticorps utilisés pour la capture et/ou la détection des antigènes sont eux choisis de telle sorte qu'ils reconnaissent précisément des épitopes non modifiés présents sur les antigènes du patient, et qu'ils ne puissent ainsi pas se lier aux antigènes modifiés, qui ne présentent plus ces mêmes épitopes. Puisque les épitopes ne sont plus identiques, il n'existe donc plus de compétition entre les anticorps utilisés pour capturer et/ou détecter l'antigène de VHC et les anticorps du patient. A l'inverse d'un certain nombre de techniques de l'art antérieur, la capture de l'antigène de la capside et celle des anticorps anti-capside pourront se faire sur une seule et même zone protéique de la capside et éviteront la perte de détection d'un certain nombre d'anticorps anti-capside, comme on le verra plus loin.

De multiples épitopes ayant été identifiés dans la partie N-terminale de la capside, cette zone protéique est la plus appropriée pour obtenir à la fois une détection très sensible de l'antigène de capside et des anticorps anti-capside.

Grâce à la présente invention, il est possible d'utiliser simultanément pour la détection des anticorps et des antigènes les séquences les plus immunoréactives et d'augmenter ainsi la sensibilité de la détection.

Il va de soit que l'invention n'est pas limitée à la détection de la seule infection due au virus de l'hépatite C (VHC). Elle englobe aussi la généralisation du procédé, des réactifs et des trousses décrits ci-après, à la détection de l'infection, et/ou du suivi de infection, due à toutes sortes de microorganismes infectieux (comme des virus, tels que, par exemple, les virus responsables des diverses hépatites A, B, C, D ou E, les rétrovirus, en particulier les rétrovirus responsables du SIDA chez l'homme (VIH-1, VIH-1 groupe O, VIH-2) ou le singe, le cytomégalovirus (CMV), les flavivirus, dont les virus de la dengue, ainsi que les bactéries, les parasites microbiens, etc.) pour lesquels une détection simultanée d'antigènes et d'anticorps est souhaitable. L'invention, plus particulièrement décrite ici pour le virus de l'hépatite C (VHC), est d'une portée très générale que l'homme du métier appréhendera aisément. L'invention est telle que définie dans les revendications 1 à 31.

### Définitions

Le terme « *virus de l'hépatite C* » ou « *VHC* » couvre ici toutes les souches, tous les types, sous-types et génotypes du virus responsable de l'hépatite C. Le procédé de l'invention vise en effet à détecter toute infection par le VHC, quels que soient son origine et son génotype. Ceci comprend en particulier les types et sous-types bien connus du virus circulant en Europe, aux Etats-Unis, au Japon, etc... (c'est à dire les 6 génotypes majeurs : 1, 2, 3, 4, 5, 6 et leurs sous-types 1 a, 1b, 3a,... ). Voir Stuyver et al. (1994) ; Bukh (1995).

Par *« virus de l'immunodéficience humaine »* ou « *VIH »,* on entend toutes les souches, tous les types, sous-types, groupes et génotypes du rétrovirus responsable du SIDA chez l'homme. Le terme VIH regroupe en particulier les VIH-1 (VIH-1 du groupe M, VIH-1 du groupe O), et les VIH-2 et leurs variants.

Dans le contexte de l'invention, un «*échantillon biologique »* est de préférence constitué par un fluide biologique, tel que du sang, plasma, sérum, de l'urine, du liquide céphalorachidien, de la salive, etc...

Le terme «*anticorps*» se réfère à tout anticorps entier ou fragment fonctionnel d'un anticorps comprenant ou consistant en au moins un site de combinaison antigénique, permettant audit anticorps de se lier à au moins un déterminant antigénique d'un composé antigénique. A titre d'exemple de fragments d'anticorps on peut citer les fragments Fab, Fab', F(ab')₂ ainsi que les chaînes scFv (Single chain variable fragment), dsFv (Double-stranded variable fragment), etc... Ces fragments fonctionnels peuvent notamment être obtenus par génie génétique.

La production d'anticorps monoclonaux ou de sérums polyclonaux monospécifiques utiles dans le cadre de l'invention relève de techniques conventionnelles, qui sont détaillées plus loin.

Par *« anticorps de capture »,* on entend un anticorps ou une partie d'anticorps, de préférence fixé sur une phase solide, qui est capable de retenir un antigène du microorganisme, par exemple du VHC ou d'un VIH, présent dans un échantillon biologique, par liaison affine.

La présence des anticorps et antigènes dans l'échantillon biologique est révélée par des « *moyens de détection ».* S'agissant de la détection de l'antigène, l'invention prévoit notamment une détection à l'aide d'au moins un *« anticorps de détection ».* Un tel anticorps de détection, marqué, est capable de se lier à l'antigène capturé, par liaison affine, en reconnaissant un site épitopique, différent de celui reconnu par l'anticorps de capture, ou identique du fait de la présence de motif répétitif au niveau de la capside. S'agissant de la détection des anticorps, on peut utiliser notamment des anticorps anti-immunoglobuline, ou anti-isotype, marqués, par exemple des anti-immunoglobulines G.

Le *terme « marqué »* se réfère aussi bien à un marquage direct (par le biais d'enzymes, radioisotopes, fluorochromes, composés luminescents, etc) qu'à un marquage indirect (par exemple, par le biais d'anticorps eux-mêmes marqués de manière directe ou à l'aide de réactifs d'une « paire d'affinité » marquée, telle que, mais non exclusivement, la paire avidine marquée-biotine, etc.).

Par *« fragment antigénique* », on entend tout ou partie d'une protéine naturelle ou recombinée d'un microorganisme infectieux, tel que le virus de l'hépatite C ou le VIH, capable d'induire la synthèse d'anticorps chez un patient infecté ou chez un animal immunisé. Il peut notamment s'agir de tout ou partie de la protéine de capside, ou des protéines non-structurales du VHC, en particulier NS3 et NS4, qu'elles soient obtenues par génie génétique ou non. Il peut également s'agir de tout ou partie d'une protéine codée par le gène gag d'un VIH, en particulier la P25 (VIH-1) ou la P26 (VIH-2), ou d'une protéine codée par le gène d'enveloppe, notamment la gp41 (VIH-1) ou la gp36 (VIH-2).

Par *« antigène de capture* », on entend un fragment antigénique isolé, de préférence fixé sur une phase solide, qui est capable d'être reconnu par des anticorps dirigés contre le microorganisme, tels que des anticorps anti-VHC ou anti-VIH et de permettre une liaison affine avec ces derniers.

Par *« antigène de détection »,* on entend un antigène marqué et modifié comme l'antigène de capture (i.e. il contient au moins un site épitopique ou épitope détruit). Il permet soit de détecter par compétition l'antigène capturé, soit de détecter des anticorps par méthode sandwich antigène-anticorps-antigène classique, encore appelée méthode « sandwich double antigène » (Maiolini et al. (1978)).

Conformément à la présente invention, l'antigène de capture, et/ou l'antigène de détection éventuellement utilisé, en particulier en détection d'anticorps par sandwich antigène-anticorps-antigène, contient au moins un site épitopique ou épitope détruit. Un « *site épitopique »* ou « *épitope »* est une séquence d'acides aminés qui est reconnue par un anticorps et permet la liaison spécifique de celui-ci.

En ce qui concerne les protéines du VHC, plusieurs épitopes de la protéine de capside ont été identifiés. On connaît notamment les épitopes localisés entre l'acide aminé 16 et l'acide aminé 40, et entre l'acide aminé 44 et l'acide aminé 47. On peut se référer aussi, par exemple, aux articles ou divulgations Okamoto et al. (1990) ; Nasoff et al. (1991) ; Leahy et al. (1991) ; Takahashi et al. (1992) ; Sällberg et al. (1992) ; et Ishida, (1993).

De nombreux épitopes de protéines non structurales du VHC sont connus aussi de l'homme du métier. Sur la protéine NS3, on connaît les épitopes localisés entre l'acide aminé 1188 et l'acide aminé 1493 (Yang et al. (1995)), entre l'acide aminé 1175 et l'acide aminé 1334 (Yang et al. (1999)), et un épitope entre l'acide aminé 1460 et l'acide aminé 1532 (Clayes et al. (1995)).

L'un des épitopes NS4 les plus connus, l'épitope 5-1-1 (AA 1689-1706), est cité dans Cerino et al. (1991).

Pour ce qui est du VIH, plusieurs épitopes ont également été décrits dans l'art antérieur. Il s'agit notamment d'épitopes de la protéine P25 du VIH-1 (groupe M) localisés entre les acides aminés 293 à 350, mais également de l'épitope immunodominant de la gp41 décrit par Gnann et al. (1987) ou un variant de cette séquence; il s'agit encore d'un épitope de la protéine P26, en particulier un épitope de séquence homologue à celle de l'épitope de la P25 du VIH-1 décrit ci-dessus, par exemple, ou d'un épitope de la gp36 du VIH-2, en particulier l'épitope immunodominant de la gp36 décrit par Gnann et al. (1987), ou un variant de cette séquence.

Par site épitopique ou épitope « *détruit* », on entend que celui-ci est modifié dans sa structure (primaire, secondaire, tertiaire et/ou quaternaire) de manière à ce que l'antigène de capture, ou de détection, qui comprend cet épitope détruit ne puisse pas se lier à l'anticorps de capture, et/ou à l'anticorps de détection, dirigé contre l'antigène détecté simultanément, tout en conservant la capacité de lier les anticorps dirigés contre le microorganisme, tels que les anticorps anti-VHC ou anti-VIH, éventuellement présents dans l'échantillon biologique, par reconnaissance d'autres sites épitopiques restés intacts. Par voie de conséquence, les anticorps de capture et/ou de détection sont choisis de manière à reconnaître spécifiquement l'épitope en question sous sa forme « intacte », c'est-à-dire « non détruite » ou « native», sur l'antigène présent dans l'échantillon biologique. Dans le cadre de la présente invention, cet épitope sous sa forme intacte est également appelée épitope "*homologue*" de l'épitope détruit.

Le terme «*spécifiquement*», quand il se réfère à une reconnaissance ou une liaison spécifique d'un anticorps pour un antigène, signifie que l'anticorps interagit avec l'antigène sans interaction substantielle avec d'autres antigènes, ou si l'on parle de reconnaissance « spécifique » avec un épitope, par reconnaissance quasi-exclusive de cet épitope. Des constantes d'association supérieures à 10⁸ L.mol⁻¹ sont préférables.

### Anticorps de capture et de détection

Les anticorps utilisés dans la présente invention sont des anticorps spécifiques de l'antigène, et, pour cette raison, sont des anticorps monoclonaux ou des anticorps polyclonaux monospécifiques, c'est à dire qu'ils ne reconnaissent spécifiquement qu'un épitope.

Les anticorps monoclonaux peuvent être obtenus selon la méthode classique de fusion lymphocytaire et culture d'hybridomes décrite par Köhler et Milstein, (1975). D'autres méthodes de préparation d'anticorps monoclonaux sont également connues (Harlow et al. (1988)). Les anticorps monoclonaux peuvent être préparés en immunisant un mammifère (par exemple une souris, un rat, un lapin, voire un être humain, etc...) et en utilisant la technique de fusion lymphocytaire conduisant à des hybridomes (Köhler et Milstein, 1975).

Des techniques alternatives à cette technique usuelle existent. On peut, par exemple, produire des anticorps monoclonaux par expression d'un acide nucléique cloné à partir d'un hybridome. On peut également produire des anticorps par la technique d'expression sur phage (« phage display »), en introduisant des ADNc d'anticorps dans des vecteurs, qui sont typiquement des phages filamenteux (par exemple, fUSE5 pour *E.coli*, Scott et al. (1990)). Ces derniers constituent des banques et présentent des fragments scFv à leur surface. Des protocoles de construction de ces banques d'anticorps sont décrits dans Marks et al. (1991).

Les anticorps polyclonaux peuvent être obtenus à partir du sérum d'un animal immunisé contre un antigène de nature peptidique selon les modes opératoires usuels.

D'une manière générale, on peut utiliser, par exemple, comme immunogène, un polypeptide, en particulier recombiné, ou un oligopeptide. Selon un protocole classique, des lapins sont immunisés avec l'équivalent de 1mg de l'immunogène peptidique selon la procédure décrite par Benoit et al. (1982). A intervalles de quatre semaines, les animaux sont traités par des injections de 200 µg d'antigène et saignés 10 à 14 jours plus tard. Après la troisième injection, on évalue la capacité de l'anti-sérum à se lier au peptide antigène radiomarqué à l'iode, préparé par la méthode chloramine-T. On le purifie ensuite par chromatographie sur colonne échangeuse d'ions constituée de carboxyméthyl cellulose (CMC). Les molécules d'anticorps recueillies par élution sont ensuite ajustées à la concentration souhaitée par des méthodes bien connues de l'homme du métier, par exemple, en utilisant du DEAE Sephadex pour obtenir la fraction IgG.

Afin d'augmenter la spécificité du sérum polyclonal, les anticorps peuvent être purifiés par une chromatographie d'immuno-affinité (ou d'immunoadsorption) en utilisant des peptides (tels que les peptides de capside AA 16-44 et AA 39-74 du VHC, à titre d'exemple non limitatif) ayant servi à l'immunisation et immobilisés en phase solide. L'antisérum est mis en contact avec un tel peptide immobilisé en phase solide pendant une durée suffisante de façon à faire immuno-réagir le peptide avec la molécule d'anticorps afin de former un complexe immunologique en phase solide.

Les inventeurs ont ainsi plus particulièrement utilisé les anticorps anti-VHC spécifiques indiqués dans le Tableau 1 ci-après.

**Tableau 1**

| **Anticorps Monoclonal** | **Classe** | **Epitope naturel reconnu** |
|---|---|---|
| Acm 1 | IgG2a | ⁴⁴LGVR⁴⁷ (SEQ ID No.19) |
| Acm 2 | IgG2a | ³⁰IVGGVYL³⁶ (SEQ ID No.20) |
| Acm 3 | IgG1 | ²⁹QIVGGV³⁴ (SEQ ID No.21) |
| Acm 4 | IgG1 | ²⁹QIVGGV³⁴ (SEO ID No.21) |
| Acm 5 | IgG1 | ³⁰IVGGVYL³⁶ (SEQ ID No.20) |
| Acm 6 | IgG1 | ³⁰IVGGVYL³⁶ (SEQ ID No.20) |

Pour ce qui concerne le test Combo pour la détection du VIH, l'anticorps utilisé est préférentiellement un anticorps monoclonal reconnaissant une des séquences polypeptidiques suivantes, ou la séquence correspondante d'une souche VIH variante :
- épitope de la protéine P25 d'un VIH-1 du groupe M de séquence 308QASQEVKNWMTETLL322 (SEQ ID No.24) ;
- épitope de la protéine P26, en particulier un épitope de séquence homologue de celle de l'épitope de la P25 du VIH-1 décrit par l'identificateur de séquence SEQ ID No.24, comme par exemple un épitope contenant la séquence QTDPAVKNWMTQTLL (SEQ ID No.25) (isolat VIH-2: ROD).

Il va de soi qu'il est à la portée de l'homme du métier de produire ou de se procurer des anticorps monoclonaux, de spécificité épitopique similaire ou identique à celle décrite pour les anticorps ci-dessus, et qui conviennent à la mise en oeuvre de la présente invention.

### Antigènes de capture et/ou de détection

L'antigène de capture, et/ou l'antigène de détection, utilisé dans l'invention contient au moins un site épitopique détruit. La destruction d'au moins deux sites sur l'antigène peut être nécessaire, lorsque deux anticorps différents, qui risquent tous deux d'interagir avec l'antigène de capture et/ou l'antigène de détection, sont utilisés. C'est le cas, par exemple, pour les immunoessais de type sandwich, qui mettent en oeuvre un anticorps de capture immobilisé sur la même surface que l'antigène de capture, et un anticorps de détection. L'anticorps de capture est alors choisi de manière à ce qu'il reconnaisse spécifiquement un épitope, sur l'antigène naturel du patient, homologue de l'un des deux épitopes détruits sur l'antigène de capture, tandis que l'anticorps de détection est choisi de manière à ce qu'il reconnaisse spécifiquement un épitope, sur l'antigène naturel du patient, homologue de l'autre des deux épitopes détruits sur l'antigène de capture.

L'antigène de capture et/ou l'antigène de détection, peut comprendre, ou consister en un peptide mimant tout ou partie d'un épitope issu d'une protéine antigénique du microorganisme, en particulier du VHC ou d'un VIH.

De préférence, il s'agit d'un peptide de la protéine de capside, la détection des anticorps anti-capsides étant particulièrement avantageuse, notamment dans le cas d'hépatites chroniques, où on observe très peu d'antigènes, et davantage des anticorps anti-capside circulants. Il peut également s'agir de peptide(s) d'une protéine non-structurale, telle que la NS3 et/ou la NS4. Divers antigènes de capture, ou de détection, peuvent être également combinés ensemble. Ce mode de réalisation, qui met en oeuvre plusieurs antigènes de capture, et/ou de détection, différents, permet, par exemple, la détection simultanée d'anticorps anti-capside et d'anticorps anti-protéines non structurales NS3 et/ou NS4 du VHC. Une détection simultanée des anticorps anti-capside et des anticorps anti-NS3 est particulièrement préférée. L'invention comprend également une détection simultanée d'antigène capsidique, d'anticorps anti-capside, d'anticorps anti-protéines structurales d'enveloppe E1 et/ou E2, d'antigène d'enveloppe E1 et/ou E2, et/ou d'anticorps anti-protéines non structurales NS3 et/ou NS4 du VHC. D'autres combinaisons envisageables font aussi partie de l'invention.

Pareillement, la détection simultanée d'antigène gag, d'anticorps anti-gag, et/ou d'anticorps anti-enveloppe de virus du SIDA (VIH-1, VIH-2, etc..) dont les séquences ont été publiées dans les articles Wain-Hobson et al. (1985) ; Ratner et al. (1985) ; Sanchez-Pescador et al. (1985) ; Guyader et al. (1987) fait aussi partie de l'invention. Préférentiellement, l'antigène gag à détecter est la protéine P25 du VIH-1 ou la P26 du VIH-2 et les anticorps anti-gag recherchés sont dirigés contre ces protéines. Toujours préférentiellement, les antigènes d'enveloppe pour détecter les anticorps anti-enveloppe du VIH-1 et du VIH-2 sont la gp41 du VIH-1 et la gp36 du VIH-2.

Similairement, la détection d'antigène NS1, décrite par Alcon et al., (2002) simultanément à celle d'anticorps anti-NS1, voire aussi d'anticorps anti-NS2, NS3, et/ou S4 des virus de la dengue fait aussi partie de l'invention.

Les techniques d'application sont bien connues de l'homme du métier.

De manière préférentielle, car plus pratique, les antigènes de capture, et/ou de détection, sont des peptides synthétiques produits par les techniques standards bien connues de l'homme du métier. On peut citer comme exemple la synthèse de type Merrifield qui est avantageuse pour des raisons de pureté, de spécificité antigénique, d'absence de produits secondaires non désirés et pour sa facilité de mise en oeuvre (Merrifield, (1963); R.C.Sheppard (1971) ; Atherton et al. (1989)). Comme synthétiseur automatique, on peut utiliser le synthétiseur "9050 Plus Pep Synthesizer" de Millipore, le synthétiseur "Pioneer" de Perseptive, le synthétiseur "433A" de ABI (Applied Biosystems Inc.) ou le synthétiseur « Symphony » de Rainin. Les peptides peuvent également être obtenus par synthèse en phase homogène.

La destruction des épitopes sur ces antigènes de capture, et/ou de détection, peut être réalisée de diverses manières. La seule condition requise à cette modification des sites épitopiques est que l'antigène de capture, et/ou de détection, ne puisse pas se lier à l'anticorps de capture, et/ou à l'anticorps de détection, tout en conservant substantiellement la capacité de lier les anticorps dirigés contre le microorganisme, en particulier des anticorps anti-VHC ou anti-VIH, éventuellement présents dans l'échantillon biologique.

Une modification d'au moins un acide aminé, de préférence deux acides aminés, dans chaque site épitopique visé, peut suffire à détruire l'épitope, sans toutefois déstabiliser les autres épitopes non modifiés. Une telle modification peut être notamment obtenue par une substitution d'acide aminé, par exemple en substituant un résidu non-glycine par un résidu glycine ou alanine, ou en substituant un acide aminé d'une classe par un acide aminé d'une autre classe. On peut, par exemple, substituer un acide aminé à chaîne latérale polaire (tel que l'asparagine, la glutamine, la sérine, la thréonine et la tyrosine), par un acide aminé à chaîne latérale non polaire (tel que la glycine, l'alanine, la valine, la leucine, l'isoleucine, la phénylalanine, la méthionine, le tryptophane et la cystéine), et vice versa. On peut également substituer un acide aminé à chaîne latérale basique (tel que la lysine, l'arginine et l'histidine), par un acide aminé à chaîne latérale acide (tel que l'acide aspartique et l'acide glutamique), et vice versa, ou encore substituer un acide aminé dont la chaîne latérale porte un cycle (par exemple la tyrosine, la phénylalanine), par un acide aminé à chaîne latérale linéaire, et vice versa. Une délétion d'un ou plusieurs acides aminés, ou une insertion d'un ou plusieurs acides aminés, en particulier d'acides aminés non naturels, dans l'épitope, permet également de détruire l'épitope. Des modifications des groupes fonctionnels des acides aminés, à savoir notamment les groupes OH, NH₂, ou SH, sont également possibles.

Parmi les peptides utiles dans le cadre de la présente invention, on peut concevoir des peptides dérivés de toutes sortes de microorganismes infectieux (comme les virus, tels que, par exemple, les virus responsables des diverses hépatites, les rétrovirus, en particulier les rétrovirus responsables du SIDA (VIH-1, VIH-2,..), le virus CMV, les virus de la dengue, ainsi que les bactéries, les parasites microbiens, etc..) pour lesquels une détection simultanée d'antigènes et d'anticorps est souhaitable.

En particulier, et à titre d'illustration et non exclusif, on peut citer notamment les peptides de capside du VHC, plus particulièrement ceux allant des acides aminés 1 à 75, 6 à 68 et 1 à 53. Ces séquences, qui correspondent à des séquences consensus du génotype 1 (sous-types 1a, 1b, 1c) sont présentées dans la liste de séquences annexée et désignées respectivement SEQ ID No.1, No.2 et No.3 :

De la même manière, un peptide ou polypeptide utile dans le cadre de la présente invention est un polypeptide gag ou d'enveloppe d'un VIH.

Il s'agit en particulier d'un peptide correspondant à la séquence consensus de la protéine P25 du VIH-1 dont la séquence est désignée par l'identificateur de séquence SEQ ID No. 23 dans la liste de séquences annexée.

Il peut s'agir également d'un peptide comprenant un épitope de la gp41 d'un VIH-1, notamment un peptide de séquence SEQ ID No.26 à SEQ ID No.31 :
SEQ ID No.26 : LGLWGCSGKLIC,
SEQ ID No.27 :LGIWGCSGKLIC,
SEQ ID No.28 : LGLWGCSGKHIC,
SEQ ID No.29 : LGMWGCSGKHIC
SEQ ID No.30 : RILAVERYLKDQQLLGIWGCSGKLIC
SEQ ID No.31 : RILAVERYLKDQQLLGIWGSGKLICTTAVPWNAS.

L'utilisation d'un polypeptide gag ou d'enveloppe d'un VIH-2 comme antigène de capture et/ou de détection fait également partie de la présente invention. Il s'agit en particulier d'un polypeptide de la gp36, de séquence SEQ ID No.32 ou SEQ ID No.33 tel que suit :
SEQ ID No.32 : LNSWGCAFRQVC,
SEQ ID No.33 : RVTAIEKYLQDQARLNSWGCAFRQVCHTTVPWVNDS.

Il peut aussi s'agir d'un polypeptide de la protéine P26 d'un VIH-2, par exemple un polypeptide de séquence homologue à celle du polypeptide de la protéine P25 décrit par l'identificateur de séquence SEQ ID No.23.

Font également partie de l'invention des peptides ou polypeptides modifiés, utiles comme antigènes de capture, et/ou de détection. Il s'agit en particulier de fragments peptidiques de capside, portant au moins un site épitopique modifié. Un objet de l'invention est donc un peptide ou polypeptide issu de la protéine de capside du VHC, portant au moins un site épitopique intact et au moins un site épitopique détruit, ledit site épitopique détruit étant ainsi rendu incapable d'être reconnu par un anticorps anti-capside. Des peptides préférés présentent une substitution de deux, trois ou quatre acides aminés, notamment dans la partie constituée par les acides aminés 20 à 40 et dans la partie constituée par les acides aminés 44 à 47. Les mutations suivantes sont particulièrement intéressantes et préférées :
- substitution des acides aminés 34, 44, et 47 par des résidus glycine (séquence SEQ ID No.4, peptide désigné « Cap 1-75 (G34-G44-G47) » ) :
- substitution des acides aminés 31, 44, et 47 par des résidus glycine (séquence SEQ ID No.5, peptide désigné «Cap 1-75 (G31-G44-G47) »);
- substitution des acides aminés 36, 44, et 47 par des résidus glycine (séquence SEQ ID No.6, peptide désigné « Cap 1-75 (G36-G44-G47) ») ;
- substitution des acides aminés 34, 44, et 47 par des résidus glycine (séquence SEQ ID No.7, peptide désigné « Cap 6-68 (G34-G44-G47)» ) ;
- substitution des acides aminés 31, 44, et 47 par des résidus glycine (séquence SEQ ID No.8, peptide désigné « Cap 6-68 (G31-G44-G47)» ) ;
- substitution des acides aminés 36, 44, et 47 par des résidus glycine (séquence SEQ ID No.9, peptide désigné « Cap 6-68 (G36-G44-G47)» ) ;
- substitution des acides aminés 34, 44, et 47 par des résidus glycine (séquence SEQ ID No.10, peptide désigné « Cap 1-53 (G34-G44-G47) » ) ;
- substitution des acides aminés 31, 44, et 47 par des résidus glycine (séquence SEQ ID No.11, peptide désigné « Cap 1-53 (G31-G44-G47)» ) ;
- substitution des acides aminés 36, 44, et 47 par des résidus glycine (séquence SEQ ID No.12, peptide désigné « Cap 1-53 (G36-G44-G47)» ) ;
- délétion des acides aminés 45, et 46 :
- modification de longueur du squelette : où βA représente la β-Alanine
- modification de longueur du squelette par insertion d'acide aminé et mutations:
- inversion de polarité : où nL représente la norLeucine
- substitution d'acides aminés
- substitution d'acides aminés où hS représente l'homoSérine

L'invention a également trait à un peptide ou polypeptide issu d'une protéine gag d'un VIH, portant au moins un site épitopique intact et au moins un site épitopique détruit, ledit site épitopique détruit étant ainsi rendu incapable d'être reconnu par un anticorps dirigé contre la même protéine gag. Plus particulièrement ledit polypeptide comprend une séquence consensus de la protéine P25 du VIH-1 et peut présenter une substitution d'un, deux, trois, quatre ou cinq acides aminés, notamment dans la partie constituée par les acides aminé 293 à 322. Préférentiellement, ledit polypeptide modifié présente la séquence consensus de la protéine P25 du VIH-1 (M) dans laquelle les acides aminés 295, 298, 310, et 312 ont été substitués par un résidu glycine, et l'acide aminé 316 par un résidu phénylalanine :

Lors de l'utilisation de ces séquences peptidiques, il peut être avantageux d'ajouter en position N-terminale les acides aminés C-G-G- (i.e. *Cys-Gly-Gly*-) afin de pouvoir plus facilement les accrocher à un support ou toute molécule d'intérêt. Le peptide C-G-G-Cap 1-75 (G34-G44-G47) est particulièrement avantageux à cet égard.

### Procédés de détection

L'invention fournit de manière générale un procédé de détection *in vitro* d'une infection par un microorganisme dans un échantillon biologique, comprenant la détection simultanée d'un antigène dudit microorganisme et d'un anticorps dirigé contre ledit microorganisme, présents dans un échantillon biologique, le procédé étant tel que défini dans la revendication 1.

Il est entendu que le procédé de l'invention n'est pas restreint à l'utilisation d'un seul couple anticorps/antigène. Il peut être réalisé en mettant en oeuvre plusieurs anticorps de capture différents, et plusieurs antigènes de capture différents.

En particulier, l'invention fournit donc un procédé de détection *in vitro* d'une infection par le virus de l'hépatite C (VHC) dans un échantillon biologique, comprenant la détection simultanée d'un antigène du VHC et d'un anticorps anti-VHC, dirigé contre ledit antigène du VHC, présents dans un échantillon biologique, procédé comprenant
a) la mise en présence de l'échantillon biologique avec un anticorps de capture anti-VHC et un antigène de capture du VHC ;
b) l'incubation du mélange dans des conditions permettant la formation de complexes antigènes-anticorps ;
c) la révélation des complexes antigènes-anticorps formés, qui met en oeuvre un anticorps de détection, marqué, capable de se lier à l'antigène du VHC capturé, et/ou éventuellement un antigène de détection, marqué, capable de se lier à l'anticorps anti VHC capturé ;
et dans lequel l'antigène de capture du VHC comprend, ou est, un fragment antigénique du VHC dont au moins un épitope est détruit;
et l'anticorps de capture et/ou de détection reconnaît ledit au moins un épitope, intact, de l'antigène capturé.

L'invention propose en outre un procédé de détection in vitro d'une infection par un virus de l'immunodéficience humaine (VIH) dans un échantillon biologique, comprenant la détection simultanée d'un antigène d'un VIH et d'un anticorps anti-VIH, dirigé contre ledit antigène du VIH, présents dans un échantillon biologique, procédé comprenant
a) la mise en présence de l'échantillon biologique avec un anticorps de capture anti-VIH et un antigène de capture d'un VIH;
b) l'incubation du mélange dans des conditions permettant la formation de complexes antigènes-anticorps ;
c) la révélation des complexes antigènes-anticorps formés, qui met en oeuvre un anticorps de détection, marqué, capable de se lier à l'antigène du VIH capturé, et/ou éventuellement un antigène de détection, marqué, capable de se lier à l'anticorps anti-VIH capturé ;
et dans lequel l'antigène de capture du VIH comprend, ou est, un fragment antigénique du VIH dont au moins un épitope est détruit;
et l'anticorps de capture et/ou de détection reconnaît ledit au moins un épitope, intact, de l'antigène capturé.

Préférentiellement ledit procédé permet la détection d'un VIH-1, la détection d'un VIH-2, ou la détection à la fois d'un VIH-1 ou d'un VIH-2. En conséquence, selon l'alternative considérée, l'antigène de capture du VIH utilisé peut comprendre ou être un antigène de capture du VIH-1, un antigène de capture du VIH-2. et un antigène de capture du VIH-1 et du VIH-2 ou une combinaison d'un antigène de capture du VIH-1 et d'un antigène de capture du VIH-2, respectivement

L'échantillon biologique peut être éventuellement traité dans une étape préalable, ou mis en présence de l'antigène de capture et de l'anticorps de capture dans des conditions favorisant l'exposition des antigènes à détecter. Avantageusement, on traite l'échantillon par un agent dénaturant, avant la détection, et de préférence, avant sa mise en contact avec les anticorps utilisés. Dans le cas de la détection de la capside du VHC, ou de la protéine gag d'un VIH, cet agent dénaturant peut notamment être constitué d'un ou plusieurs détergents de type non-ionique, tel(s) que, par exemple, le Nonidet P-40 (NP40) (tert-Octylphenoxy poly(oxyethylene) ethanol, encore appelé IGEPAL CA630), ou encore d'une solution acide.

Cet immunoessai combiné peut être réalisé selon divers formats bien connus de l'homme du métier : en phase solide; en un temps ou en deux temps ; en méthode double sandwich (sandwich pour les deux détections d'antigènes et d'anticorps) ; ou en méthode indirecte (pour la détection d'anticorps) combinée à une méthode sandwich (pour la détection d'antigène).

Selon l'invention, l'anticorps de capture et l'antigène de capture sont immobilisés sur une phase solide. On peut utiliser, à titre d'exemples non limitatifs de phase solide, des microplaques, en particulier des microplaques de polystyrène, telles que celles commercialisées par la société Nunc, Danemark. On peut également utiliser des particules ou des billes solides, des billes paramagnétiques, telles que celles fournies par Dynal ou Merck-Eurolab (France) (sous la marquée Estapor^{™}), ou encore des tubes à essai en polystyrène ou polypropylène, etc...

Un format d'immunoessai de type sandwich entre deux anticorps (de capture et de détection) est particulièrement avantageux pour la détection des antigènes présents dans l'échantillon biologique, tandis que les anticorps peuvent être révélés en mettant en oeuvre un antigène de capture et un conjugué marqué qui se fixe sur l'anticorps (selon un format communément désigné comme « format indirect »), par exemple la proteine A marquée ou encore un anticorps anti immunoglobuline, ou anti-isotype, marqué. On peut encore détecter avantageusement les anticorps en mettant en oeuvre un antigène de capture et un antigène marqué qui se fixent sur l'anticorps (selon un format désigné comme « sandwich antigène-anticorps-antigène » ou « sandwich double antigène »).

D'une manière générale, selon l'invention, l'anticorps de capture et l'anticorps de détection (notamment utilisé dans le cas du sandwich) sont choisis de sorte qu'ils reconnaissent l'antigène cible naturel à détecter, présent dans l'échantillon biologique, en un épitope, intact, homologue de l'épitope détruit sur l'antigène de capture et qu'ils ne reconnaissent pas l'épitope détruit sur l'antigène de capture.

La détection simultanée d'antigène d'un microorganisme et d'anticorps dirigé contre ledit microorganisme, notamment la détection simultanée de l'antigène de VHC et des anticorps anti-VHC ou encore de l'antigène d'un VIH et des anticorps anti-VIH, selon l'invention, peut être réalisée en un seul temps, à savoir en mettant simultanément en présence l'échantillon biologique, les moyens de détection, tels que notamment le ou les anticorps de détection, en même temps que le ou les anticorps de capture et le ou les antigène(s) de capture. Dans ce cas, immunoessai de détection de l'antigène et l'immunoessai de détection des anticorps sont tous les deux réalisés de préférence en sandwich. De manière alternative, les moyens de détection, tels que notamment le ou les anticorps de détection, peuvent être ajoutés au mélange dans un deuxième temps, c'est à dire après que les premiers complexes antigènes-anticorps se sont formés. On parle alors d'essai en deux temps.

Comme cela est décrit ci-dessus, l'antigène de capture peut être notamment un fragment antigénique de la protéine de capside du VHC, dans lequel au moins un site épitopique est détruit. L'antigène de capture peut être également constitué par un fragment antigénique d'une protéine non structurale du VHC, notamment mais non exclusivement de la NS3 ou de la NS4, dans lequel au moins un site épitopique est aussi détruit. Enfin, l'antigène de capture peut être également constitué par une combinaison d'un fragment antigénique de la protéine de capside, dans lequel au moins un site épitopique est détruit, et d'un fragment antigénique d'une protéine non structurale du VHC, NS3 ou NS4, dans lequel au moins un site épitopique est aussi détruit. Toute variante envisageable par l'homme du métier fait partie de l'invention.

Selon un mode de réalisation préféré de l'invention, le procédé de détection d'une infection par le virus de l'hépatite C (VHC) dans un échantillon biologique comprend :
a) la mise en présence de l'échantillon avec un anticorps de capture du VHC et un antigène de capture du VHC fixés sur une phase solide;
b) l'incubation du mélange dans des conditions permettant la formation de complexes antigènes-anticorps ;
c) la séparation de la phase solide et de la phase liquide ;
d) la mise en contact de la phase solide avec un anticorps de détection, marqué, capable de lier l'antigène de VHC capturé, et un ou des anticorps anti-immunoglobuline, ou anti-isotype, marqué, capable de lier l'anticorps anti-VHC capturé,
l'antigène de capture d'anticorps anti-VHC comprenant ou étant un fragment antigénique de la protéine de capside du VHC, dont deux épitopes ont été détruits,
et les anticorps de capture et de détection reconnaissant chacun un desdits épitopes, intacts, de l'antigène de capside capturé.

Selon un autre mode de réalisation, le procédé de l'invention permet la détection d'une infection par un VIH, tel qu'un VIH-1 ou un VIH-2. Préférentiellement ledit procédé comprend à la fois un antigène de capture dérivant d'un VIH-1 et un antigène de capture dérivant d'un VIH-2 de manière à pouvoir détecter à la fois des anticorps anti-VIH-1 et anti-VIH2. Selon ce mode préférentiel, le procédé selon l'invention peut donc être utilisé indistinctement pour détecter une infection par le VIH-1 et/ou le VIH-2.

L'antigène de capture comprend au minimum un fragment antigénique d'une protéine gag d'un VIH, par exemple de la protéine P25 du VIH-1 ou de la P26 du VIH-2, dans lequel au moins un site épitopique est détruit. L'antigène de capture peut en outre comprendre un fragment antigénique d'une protéine d'enveloppe d'un VIH, notamment mais non exclusivement de la gp41 du VIH-1 ou de la gp36 du VIH-2. Enfin, l'antigène de capture peut être constitué par une combinaison de fragments antigéniques d'une protéine gag du VIH-1 et du VIH-2, complétée le cas échéant de fragments antigéniques d'une protéine de l'enveloppe du VIH-1 et du VIH-2, Toute variante envisageable par l'homme du métier fait partie de l'invention.

Selon un mode de réalisation préféré de l'invention, le procédé de détection d'une infection par un virus de l'immunodéficience humaine (VIH) dans un échantillon biologique comprend :
a) la mise en présence de l'échantillon avec un anticorps de capture du VIH et un antigène de capture du VIH fixés sur une phase solide;
b) l'incubation du mélange dans des conditions permettant la formation de complexes antigènes-anticorps ;
c) la séparation de la phase solide et de la phase liquide ;
d) la mise en contact de la phase solide avec un anticorps de détection, marqué, capable de lier l'antigène de VIH capturé, et un ou des anticorps anti-immunoglobuline, ou anti-isotype, marqué, capable de lier l'anticorps anti-VIH capturé,
l'antigène de capture d'anticorps anti-VIH comprenant, ou étant, un fragment antigénique de la protéine gag du VIH, dont au moins un épitope a été détruit,
et les anticorps de capture et de détection reconnaissant chacun un desdits épitopes, intacts, de l'antigène gag capturé.

Préférentiellement, le procédé selon l'invention vise la détection d'une infection soit par le VIH-1, soit par le VIH-2, soit par le VIH-1 et le VIH-2. Selon cette dernière alternative, l'antigène de capture d'anticorps anti-VIH est la combinaison d'un fragment de la protéine gag du VIH-1 et d'un fragment de la protéine gag du VIH-2.

Dosages ELISA, radioimmunoessais, ou toute autre technique de détection peuvent être mises en oeuvre pour révéler la présence des complexes antigènes-anticorps formés. Un même type ou plusieurs types de marqueurs peuvent être utilisés pour détecter, d'une part, l'antigène de microorganismes infectieux, notamment antigène VHC ou VIH, et, d'autre part, l'anticorps dirigé contre le microorganisme infectieux, notamment l'anticorps anti-VHC ou anti-VIH.

La détection de la présence d'antigènes ou d'anticorps dans l'échantillon biologique peut être complétée par une quantification, par exemple par mesure des signaux émis par les marqueurs (couleur, luminescence, radioactivité,...), selon les techniques standards bien connues de l'homme de métier.

### Trousses

Les trousses et réactifs utiles pour la détection d'une infection par un microorganisme, tel que le virus de l'hépatite C (VHC) ou un virus de l'immunodéficience humaine (VIH), dans un échantillon biologique, conformément au procédé de l'invention, peuvent étre fournis pour une mise en pratique de invention facile et applicable à de nombreux échantillons biologiques.

L'invention concerne aussi une trousse utile pour la détection d'une infection par un virus de l'hépatite C (VHC) ou un virus de l'immunodéficience humaine (VIH) dans un échantillon biologique, comprenant :
- un antigène de capture, immobilisé sur une phase solide, qui comprend un fragment antigénique d'une protéine dudit VHC ou VIH, ledit fragment comprenant au moins un épitope détruit, étant rendu incapable d'être reconnu par un anticorps de capture et un anticorps de détection dirigés contre ledit au moins un épitope, intact, sélectionné dans le groupe d'épitopes de séquences suivantes ⁴⁴LGVR⁴⁷ (SEQ ID No.19), ³⁰IVGGVYL³⁶ (SEQ ID No.20), ²⁹QIVGGV³⁴ (SEQ ID No.21), QASQEVKNWMTETLL (SEQ ID No.24) et QTDPAVKNWMTQTLL (SEQ ID No.25), tout en conservant la capacité de lier les anticorps dirigés contre le microorganisme éventuellement présents dans l'échantillon biologique, par reconnaissance d'autres sites épitopiques restés intacts;
- ledit anticorps de détection, monoclonal ou polyclonal monospécifique, dirigé contre ledit un moins un épitope, intact, de ladite protéine du VHC ou VIH ; et
- ledit anticorps de capture monoclonal ou polyclonal monospécifique, immobilisé sur une phase solide, dirigé contre ledit au moins un épitope, intact, de ladite protéine du VHC ou VIH;
ladite protéine du VHC étant la protéine de capside du VHC et l'anticorps de capture et/ou de détection reconnaissant un épitope sélectionné dans le groupe d'épitopes de séquences suivantes : ⁴⁴LGVR⁴⁷ (SEQ ID No.19), ³⁰IVGGVYL³⁶ (SEQ ID No.20) et ²⁹QIVGGV³⁴ (SEQ ID No.21), ou
ladite protéine du VIH étant la protéine P25 du VIH-1 et l'anticorps de capture et/ou de détection reconnaissant l'épitope de séquence QASQEVKNWMTETLL (SEQ ID No.24), ou
ladite protéine du VIH étant la protéine P26 du VIH-2 et l'anticorps de capture et/ou de détection reconnaissant l'épitope de séquence QTDPAVKNWMTQTLL (SEQ ID No.25).

De manière avantageuse, cette trousse peut contenir plusieurs antigènes et plusieurs anticorps de capture.

Comme cela est décrit ci-dessus, l'anticorps de capture et l'antigène de capture sont présentés sous une forme immobilisée sur une phase solide, telle qu'une microplaque.

Une trousse préférée comprend
a) l'antigène de capture, qui comprend, ou est, un fragment antigénique d'une protéine du VHC, ledit fragment comprenant au moins deux épitopes détruits, tout en conservant la capacité de lier les anticorps anti-VHC éventuellement présents dans un échantillon biologique ;
b) un anticorps de capture dirigé contre l'un desdits épitopes, intacts, de ladite protéine du VHC;
   ledit antigène de capture et ledit anticorps de capture étant immobilisés sur une phase solide ;
c₁) un anticorps de détection, marqué, dirigé contre un autre desdits épitopes, intacts, de ladite protéine du VHC,
c₂) et/ou éventuellement un antigène de détection, marqué, qui comprend, ou est, un fragment antigénique d'une protéine du VHC, ledit fragment comprenant au moins deux épitopes détruits, tout en conservant la capacité de lier les anticorps anti-VHC éventuellement présents dans un échantillon biologique,
ladite protéine du VHC étant la protéine de capside et lesdits au moins deux épitopes, intacts, étant sélectionnés dans le groupe d'épitopes de séquences suivantes : ⁴⁴LGVR⁴⁷ (SEQ ID No.19). ³⁰IVGGVYL³⁶ (SEQ ID No.20), ²⁹QIVGGV³⁴ (SEQ ID No.21).

De manière préférentielle également, une trousse selon l'invention comprend
a) l'antigène de capture, qui comprend, ou est, un fragment antigénique d'une protéine du VIH, ledit fragment comprenant au moins un épitope détruit, tout en conservant la capacité de lier les anticorps anti-VIH éventuellement présents dans un échantillon biologique ;
b) un anticorps de capture dirigé contre l'un desdits épitopes, intacts, de ladite protéine du VIH;
   ledit antigène de capture et ledit anticorps de capture étant immobilisés sur une phase solide ;
c1) un anticorps de détection, marqué, dirigé contre un autre desdits épitopes, intacts, de ladite protéine du VIH, et/ou
c2) éventuellement, un antigène de détection, marqué, qui comprend, ou est, un fragment antigénique d'une protéine du VIH, ledit fragment comprenant au moins un épitope détruit, tout en conservant la capacité de lier les anticorps anti-VIH éventuellement présents dans un échantillon biologique,
ladite protéine du VIH étant la protéine P25 du VIH-1 et ledit au moins un épitope, intact, ayant la séquence QASQEVKNWMTETLL (SEQ ID No.24), ou ladite protéine du VIH étant la protéine P26 du VIH-2 et et ledit au moins un épitope, intact, ayant la séquence QTDPAVKNWMTQTLL (SEQ ID No.25).

La trousse peut comprendre en outre un moyen de détection desdits anticorps présents dans l'échantillon biologique et complexés audit antigène de capture, par exemple un anticorps anti-immunoglobuline, ou anti-isotype, marqué, ou encore un antigène de détection. L'antigène de détection est un fragment antigéniqur de la protéine de capside du VHC ou P25 ou P26 d'un VIH, qui comprend au moins un épitope détruit, tout en conservant la capacité de lier les anticorps (anti-VHC ou anti-VIH) éventuellement présents dans l'échantillon.

La trousse peut encore comprendre, en outre, un détergent, plus particulièrement un détergent non-ionique, tel que le NP40 (Sigma) ou tout détergent non-ionique équivalent connu de l'homme du métier.

Comme cela est décrit ci-dessus, l'antigène de capture, comme l'antigène de détection, peut être notamment un fragment de la protéine de capside du VHC, fragment dont au moins un site épitopique est détruit, et l'anticorps de capture et/ou de détection est alors choisi pour reconnaître ledit site épitopique, intact, de la protéine de capside.

En particulier, l'invention a pour objet un ensemble de réactifs comprenant ou constitué d'un peptide de capside du VHC muté en au moins deux sites épitopiques distincts, tel que le peptide 1-75 (G34-G44-G47), et d'une paire d'anticorps ne pouvant pas reconnaître le peptide 1-75 (G34-G44-G47) alors qu'ils reconnaissent la séquence native de VHC correspondante.

En particulier, l'invention a pour objet un ensemble de réactifs comprenant ou constitué d'un peptide de la protéine P25 muté en au moins un site épitopique distinct, tel que le peptide de séquence SEQ ID No.22, et d'une paire d'anticorps ne pouvant pas reconnaître le peptide de séquence SEQ ID No.22 alors qu'ils reconnaissent la séquence native de la protéine P25 du VIH-1.

Les figures et exemples suivants illustrent l'invention sans en limiter la portée.

### LEGENDE DES FIGURES :

La figure 1 est un schéma illustrant un procédé de détection du type de ceux mis en oeuvre antérieurement à l'invention, avec, fixés sur une phase solide, un anticorps de capture et un antigène de capture non modifié. Ce schéma illustre la compétition de l'anticorps du patient avec l'anticorps de détection vis-à-vis de la liaison à l'antigène de capture.
La figure 2, par comparaison, est un schéma illustrant un mode de réalisation de la présente invention. L'antigène de capture étant muté, il n'est plus capable d'être reconnu par l'anticorps de détection. Il n'y a donc plus interférence entre la détection de l'anticorps et la détection de l'antigène.

### EXEMPLES : Détection d'une infection par le virus de l'hépatite C

### Matériels pour les protocoles 1 a et 1 b:

Conjugué anticorps monoclonal anti-capside-peroxydase (« acm-POD ») : un conjugué de l'anticorps monoclonal anti-capside, Acm 2 (voir Tableau 1), marqué à la peroxydase est préparé selon le protocole décrit dans la demande de brevet EP 0 752 102. Ce conjugué anticorps Acm2 marqué à la peroxydase est dilué dans le diluant de la 1 ère étape (décrit ci-après) de sorte à permettre d'obtenir in fine, avec un échantillon positif bien documenté, une densité optique élevée (supérieure, par exemple, à 1,5 Unité) et ce, dans des conditions bien connues de l'homme du métier.

Un autre anticorps monoclonal anti-capside, Acm 1 (voir Tableau 1), est aussi utilisé pour être immobilisé sur la phase solide (voir ci-après).

### Matériels pour le protocole 1 c:

Conjugué anticorps monoclonal anti-capside-peroxydase (« acm-POD ») : un conjugué de l'anticorps monoclonal anti-capside, Acm 1 (voir Tableau 1), marqué à la peroxydase est préparé selon le protocole décrit dans la demande de brevet EP 0 752 102. Ce conjugué anticorps Acm1 marqué à la peroxydase est dilué dans le diluant de la 1ère étape (décrit ci-après) de sorte à permettre d'obtenir in fine, avec un échantillon positif bien documenté, une densité optique élevée (supérieure, par exemple, à 1,5 Unité) et ce, dans des conditions bien connues de l'homme du métier.

Un autre anticorps monoclonal anti-capside, l'anticorps monoclonal anti-capside, Acm 3 (voir Tableau 1), est aussi utilisé pour être immobilisé sur la phase solide (voir ci-après).

### Matériels pour le protocole 1d:

1) Conjugué anticorps monoclonal anti-capside-biotine : un conjugué de l'anticorps monoclonal anti-capside, Acm 5 (voir Tableau 1), marqué à la biotine est préparé selon le protocole décrit par Greg T. Hermanson en 1996. Cet anticorps Acm 5 marqué à la biotine est dilué dans le diluant de la 1ère étape (décrit ci-après) de sorte à permettre d'obtenir in fine, avec un échantillon positif bien documenté, une densité optique élevée (supérieure, par exemple, à 1,5 Unité) et ce, dans des conditions bien connues de l'homme du métier.

Un autre anticorps monoclonal anti-capside, l'anticorps monoclonal anti-capside, Acm 1 (voir Tableau 1), est aussi utilisé pour être immobilisé sur la phase solide (voir ci-après).

2) Conjugué Streptavidine marqué à la peroxydase: un conjugué de streptavidine marqué à la peroxydase est préparé selon le protocole décrit par Greg T. Hermanson en 1996. Au moment de la mise en oeuvre du protocole 1d, ce conjugué de streptavidine marquée à la peroxydase est dilué dans le diluant de la 2ème étape (décrit ci-après) de sorte à permettre d'obtenir in fine, avec un échantillon positif bien documenté, une densité optique élevée (supérieure, par exemple, à 1,5 Unité) et ce, dans des conditions bien connues de l'homme du métier.

### Matériels communs pour les protocoles 1a, 1b, 1 c et 1d :

1) Phase solide choisie: microplaque Maxisorp, Nunc (Danemark).
2) Diluants des 1 ère et 2ème étapes des protocoles selon l'invention :
   Diluant de la 1ère étape : Tampon Tris, NaCl 0,05M, à pH 6,7 additionné de NP40 ((tert-Octylphenoxy poly(oxyethylene)ethanol - IGEPAL CA 630, Sigma) à 0,25%.
   Diluant de la 2^{ème} étape : Tampon citrate (50 mM), à pH 6,7, glycérolé à 20%, contenant un conjugué anticorps polyclonal de souris anti-IgG (Fc) humaines marqué à la peroxydase (Jackson Immunoresearch Laboratories, USA) à une dilution permettant d'obtenir in fine, avec un échantillon positif bien documenté, une densité optique élevée (supérieure, par exemple, à 1,5 Unité) et ce, dans des conditions bien connues de l'homme du métier.
3) Solution de révélation: la solution de révélation était composée
   3a) d'un tampon substrat : Solution d'acide citrique (0,075M), et d'acétate de sodium (0,1M), à pH 4,0 , contenant de l'H₂O₂ à 0,015% et du Diméthylsulfoxyde (DMSO) (PROLABO) à 4%, et
   3b) d'un réactif chromogène : tetraméthylbenzidine (TMB, Sigma) à 0,7mM en concentration finale dans le tampon substrat.
4) solution d'arrét : H2SO4 1 N.

### Méthodes :

### Protocole 1a : Protocole de détection simultanée de l'antigène capside et des anticorps (anti-capside et anti-NS3, NS4) du virus de l'hépatite C dans un échantillon (sérum ou plasma)

Le principe du test est basé sur une méthode immunoenzymatique de type sandwich pour la détection de l'antigène, et de type indirect, pour la détection des anticorps.

Il repose sur les étapes suivantes :
Une solution de sensibilisation est tout d'abord réalisée:
   - avec un mélange d'antigènes VHC : un peptide muté C-G-G-Cap 1-75 (G34-G44-G47) (capside) comprenant la séquence SEQ ID No.4 et deux protéines recombinées produites par *Escherichia coli* à partir de clones sélectionnés dans les régions non structurales NS3 (clone NS3.1 : AA 1192-1492), et NS4 (clone 5-1-1 : AA 1694-1735) et
   - avec un anticorps monoclonal anti-capside (Acm 1),
en tampon Tris 0,5M, pH 7,4.

Les cupules d'une plaque de microtitrage (Nunc, Maxisorp) sont alors sensibilisées avec la solution ci-dessus à raison de 110 µl par cupule.

Les plaques de microtitrage sont mises à incuber pendant une nuit à température ambiante (18-24°C).

Après élimination de la solution de sensibilisation, les plaques sont lavées à l'aide d'un tampon phosphate (0,01 M, pH 7,4) contenant 0, 1 % de Tween 20, puis saturées par addition d'un tampon phosphate (0,01M, pH 7) contenant 5% de saccharose, 25% de lait écrémé (Candia^{™}, France, ou tout autre lait écrémé équivalent du commerce) et 10mM d'EDTA.

Dans chaque cupule, on distribue successivement 100 µl de diluant de 1ère étape, contenant l'anticorps monoclonal anti-capside Acm 2 marqué à la peroxydase, puis 50 µl d'échantillon (sérum ou plasma).

Le milieu réactionnel est mis à incuber à 37°C pendant 1,5 heure. Les antigènes de capside du VHC éventuellement présents se lient à l'anticorps monoclonal de la phase solide Acm 1 et à l'anticorps monoclonal anti-capside Acm 2 marqué à la peroxydase, formant ainsi des complexes sandwich avec ces deux anticorps. De même, si des anticorps anti-VHC sont présents, ils se lient aux antigènes fixés sur la phase solide.

Les plaques sont ensuite lavées (3 fois) à l'aide d'une solution de lavage (tampon Tris NaCl 0,01 M, pH 7,4 additionné de Tween 20 à 0,1%).

Dans chaque cupule, on distribue 100µl de diluant de 2ème étape, contenant les anticorps anti-IgG humaines marqués à la peroxydase. Le milieu réactionnel est mis à incuber à température ambiante (18-24°C) pendant 30 minutes. Les anticorps anti-IgG humaines marqués se fixent à leur tour aux anticorps spécifiques retenus sur la phase solide.

Les plaques sont ensuite lavées (5 fois) à l'aide d'une solution de lavage (tampon Tris NaCl 0,01 M, pH 7,4 additionné de Tween 20 à 0,1%). Le conjugué anti-IgG humaines non lié est ainsi éliminé.

Dans chaque cupule, on distribue 100 µl de la solution de révélation. On laisse la réaction se développer à l'obscurité pendant 30 minutes à température ambiante (18-24°C).

On distribue ensuite 100 µl de solution d'arrêt dans chaque cupule.

Après arrêt de la réaction, la lecture de la densité optique est effectuée au spectrophotomètre à 450/620 nm.

### Définition de la valeur-seuil :

La valeur seuil a été déterminée après analyse statistique des données de spécificité et sensibilité en utilisant la courbe de ROC (Receiver Operating Characteristic) (Berck et Schultz, (1986)).

L'étude de spécificité a porté sur 1000 échantillons de sujets sains et l'étude de sensibilité sur 200 échantillons positifs VHC (notamment des débuts de séroconversion) de panels commerciaux : BBI (Boston Biomedica Company, USA), Impath (USA), Serologicals (USA), Nabi (USA), ProMedDx (USA)).

La valeur-seuil est calculée pour chaque plaque à partir du signal obtenu sur un témoin positif, divisé par un coefficient X, constant, spécifique du test. Elle est d'environ 0,280 unité de DO (Densité Optique) dans les exemples présentés.

Comme on peut le constater dans ce protocole, à l'inverse de certaines techniques de l'art antérieur, la capture de l'antigène de la capside et celle des anticorps anti-capside se font, dans la présente invention, sur une seule et même zone protéique de la capside : la zone antigénique capturant les anticorps anti-capside (AA 1-75) inclut bien la zone de spécificité antigénique (AA 44-47) par laquelle l'anticorps anti-capside immobilisé (acm1) capture l'antigène de capside. Ainsi, selon l'invention, la perte de détection d'un certain nombre d'anticorps anti-capside est réduite au minimum et la sensibilité s'en trouve améliorée.

### Protocole 1b: Protocole de détection simultanée d'antigène capside et d'anticorps (anti NS3 et NS4) du virus de l'hépatite C dans un échantillon (sérum ou plasma)

Le principe du test est basé sur une méthode immunoenzymatique de type sandwich pour la détection d'antigène et de type indirect pour la détection d'anticorps.

Il repose sur les étapes suivantes :
Une solution de sensibilisation est tout d'abord réalisée:
   - avec un mélange d'antigènes VHC : deux protéines recombinées produites par *Escherichia coli* à partir de clones sélectionnés dans les régions non structurales NS3 (clone NS3.1 : AA 1192-1492), et NS4 (clone 5-1-1 : AA 1694-1735) et
   - avec un anticorps monoclonal anti-capside (Acm 1),
en tampon Tris 0,5M, pH 7,4.

Les cupules d'une plaque de microtitrage (Nunc, Maxisorp) sont alors sensibilisées avec la solution ci-dessus à raison de 110 µl par cupule.

Les étapes suivantes sont identiques à celles du protocole 1a.

### Protocole 1c: Protocole de détection simultanée d'antigène capside et d'anticorps (anti-capside et anti NS3, NS4) du virus de l'hépatite C dans un échantillon (sérum ou plasma)

Le principe du test est basé sur une méthode immunoenzymatique de type sandwich pour la détection d'antigène et de type indirect pour la détection d'anticorps.

Il repose sur les étapes suivantes :
Une solution de sensibilisation est tout d'abord réalisée:
   - avec un mélange d'antigènes VHC : un peptide muté C-G-G-Cap 1-75 (G31-G44-G47) (capside) comprenant la séquence SEQ ID No.5 ou le peptide muté C-G-G-Cap 1-53 (G31-G44-G47) comprenant la séquence SEQ ID No. 11 ou le peptide muté C-G-G-Cap 6-68 (G31-G44-G47) comprenant la séquence SEQ ID No. 8 et deux protéines recombinées produites par *Escherichia coli* à partir de clones sélectionnés dans les régions non structurales NS3 (clone NS3.1 : AA 1192-1492), et NS4 (clone 5-1-1 : AA 1694-1735) et
   - avec un anticorps monoclonal anti-capside (Acm 3),
en tampon Tris 0,5M, pH 7,4.

Les cupules d'une plaque de microtitrage (Nunc, Maxisorp) sont alors sensibilisées avec la solution ci-dessus à raison de 110 µl par cupule.

Les plaques de microtitrage sont mises à incuber pendant une nuit à température ambiante (18-24°C).

Après élimination de la solution de sensibilisation, les plaques sont lavées à l'aide d'un tampon phosphate (0,01 M, pH 7,4) contenant 0,1% de Tween 20, puis saturées par addition d'un tampon phosphate (0,01 M, pH 7) contenant 5% de saccharose, 25% de lait écrémé (Candia^{™}, France, ou tout autre lait écrémé équivalent du commerce) et 10mM d'EDTA.

Dans chaque cupule, on distribue successivement 100 µl de diluant de 1^{ère} étape, contenant l'anticorps monoclonal anti-capside Acm 1 marqué à la peroxydase, puis 50 µl d'échantillon (sérum ou plasma).

Le milieu réactionnel est mis à incuber à 37°C pendant 1,5 heure. Les antigènes de capside du VHC éventuellement présents se lient à l'anticorps monoclonal de la phase solide Acm 3 et à l'anticorps monoclonal anti-capside Acm 1 marqué à la peroxydase, formant ainsi des complexes sandwich avec ces deux anticorps. De même, si des anticorps anti-VHC sont présents, ils se lient aux antigènes fixés sur la phase solide.

Les étapes suivantes sont identiques à celles du protocole 1 a.

Comme on peut le constater pareillement ici, aussi, la capture de l'antigène de la capside et celle des anticorps anti-capside se font, dans la présente invention, sur une seule et même zone protéique de la capside : la zone antigénique capturant les anticorps anti-capside (AA 1-75) inclut bien la zone de spécificité antigénique (AA 29-34) par laquelle l'anticorps anti-capside immobilisé (acm 3) capture l'antigène de capside. Ainsi, selon l'invention, la perte de détection d'un certain nombre d'anticorps anti-capside est réduite au minimum et la sensibilité s'en trouve améliorée.

### Protocole 1d : Protocole de détection simultanée d'antigène capside et d'anticorps (anti-capside et anti-NS3, NS4) du virus de l'hépatite C dans un échantillon (sérum ou plasma)

Le principe du test est basé sur une méthode immunoenzymatique de type sandwich pour la détection de l'antigène, et de type indirect, pour la détection des anticorps.

II repose sur les étapes suivantes:
Une solution de sensibilisation est tout d'abord réalisée:
   - avec un mélange d'antigènes VHC : un peptide muté C-G-G-Cap 1-75 (G34-G44-G47) (capside) comprenant la séquence SEQ ID No.4, ou un peptide muté C-G-G-Cap 1-75 (G34-G46-G46') comprenant la séquence SEQ ID No.15 ou un peptide muté C-G-G-Cap 1-75 (G34-βA45) comprenant la séquence SEQ ID No.14 et deux protéines recombinées produites par *Escherichia coli* à partir de clones sélectionnés dans les régions non structurales NS3 (clone NS3.1 : AA 1192-1492), et NS4 (clone 5-1-1 : AA 1694-1735) et
   - avec un anticorps monoclonal anti-capside (Acm 1), en tampon Tris 0,5M, pH 7,4.

Les cupules d'une plaque de microtitrage (Nunc, Maxisorp) sont alors sensibilisées avec la solution ci-dessus à raison de 110 µl par cupule.

Les plaques de microtitrage sont mises à incuber pendant une nuit à température ambiante (18-24°C).

Après élimination de la solution de sensibilisation, les plaques sont lavées à l'aide d'un tampon phosphate (0,01M, pH 7,4) contenant 0,1 % de Tween 20, puis saturées par addition d'un tampon phosphate (0,01 M, pH 7) contenant 5% de saccharose, 25% de lait écrémé (Candia™, France, ou tout autre lait écrémé équivalent du commerce) et 10mM d'EDTA.

Dans chaque cupule, on distribue successivement 100 µl de diluant de 1^{ère} étape, contenant l'anticorps monoclonal anti-capside Acm 5 marqué à la biotine, puis 50 µl d'échantillon (sérum ou plasma).

Le milieu réactionnel est mis à incuber à 37°C pendant 1,5 heure.

Les antigènes de capside du VHC éventuellement présents se lient à l'anticorps monoclonal de la phase solide Acm 1 et à l'anticorps monoclonal anti-capside Acm 5 marqué à la biotine, formant ainsi des complexes sandwich avec ces deux anticorps.

De mêmes, si des anticorps anti-VHC sont présents, ils se lient aux antigènes fixés sur la phase solide.

Les plaques sont ensuite lavées (3 fois) à l'aide d'une solution de lavage (tampon Tris NaCl 0,01M, pH 7,4 additionné de Tween 20 à 0,1%).

Dans chaque cupule, on distribue 100µl de diluant de 2ème étape, contenant les anticorps anti-IgG humaines marqués à la peroxydase et la streptavidine marquée à la peroxydase. Le milieu réactionnel est mis à incuber à température ambiante (18-24°C) pendant 30 minutes. Les anticorps anti-IgG humaines marqués se fixent à leur tour aux anticorps spécifiques retenus sur la phase solide et la streptavidine marquée à la peroxydase se fixe à l'anticorps Acm 5 biotinylé retenu sur la même phase solide.

Les étapes suivantes sont identiques à celles du protocole 1a.

Comme on peut le constater pareillement ici, aussi, la capture de l'antigène de la capside et celle des anticorps anti-capside se font, dans la présente invention, sur une seule et même zone protéique de la capside : la zone antigénique capturant les anticorps anti-capside (AA 1-75) inclut bien la zone de spécificité antigénique (AA 44-47) par laquelle l'anticorps anti-capside immobilisé (acm1) capture l'antigène de capside. Ainsi, selon l'invention, la perte de détection d'un certain nombre d'anticorps anti-capside est réduite au minimum et la sensibilité s'en trouve améliorée.

### Exemple 1: Détection d'antigène capside du VHC dans un échantillon (sérum ou plasma), durant la fenêtre sérologique

Des échantillons sériques ou plasmatiques, prélevés chez des patients contaminés par le VHC et rassemblés dans des panels commerciaux (Impath, USA), négatifs en détection anticorps et positifs en acide nucléique (PCR), ont été testés suivant la méthode décrite au protocole 1 a.

Les résultats reportés dans le Tableau 2 sont comparés à des résultats obtenus en PCR.

**Tableau 2 :**

| **Echantillon Impath** | **Densité optique** | **Interprétation** | **Test Anticorps** | **Test PCR** |
|---|---|---|---|---|
| 1866 | 0,616 | **Positif** | Négatif | **Positif** |
| 1883 (1/2)* | 0,661 | **Positif** | Négatif | **Positif** |
| 1889 (1/2)* | 0,671 | **Positif** | Négatif | **Positif** |
| 1999 (1/2)* | 0,596 | **Positif** | Négatif | **Positif** |
| 2028 (1/2)* | 0,520 | **Positif** | Négatif | **Positif** |
| 2144 (1/2)* | 0,674 | **Positif** | Négatif | **Positif** |
| 2145 (1/2)* | 0,808 | **Positif** | Négatif | **Positif** |
| 2159 | 0,547 | **Positif** | Négatif | **Positif** |
| 1959 | 0,512 | **Positif** | Négatif | **Positif** |

| | | | | |
|---|---|---|---|---|
| * : dilution | | | | |

On constate que les résultats obtenus par la méthode selon l'invention sont directement corrélés aux résultats obtenus en PCR. L'invention décrite permet donc bien de détecter les antigènes de capside alors que les anticorps ne sont pas encore présents, c'est à dire dans des échantillons prélevés dans la fenêtre sérologique.

### Exemple 2: Détection d'anticorps anti-capside dans des échantillon (sérums ou plasmas) négatifs en antigène VHC

Des échantillons sériques ou plasmatiques, prélevés chez des patients contaminés par le VHC et rassemblés dans des panels internes et commerciaux, positifs en anticorps (capside) et négatifs en antigène (capside), ont été testés suivant les méthodes décrites dans les protocoles 1 a et 1 b.

En l'absence du peptide 1-75 (G34-G44-G47) (comprenant la SEQ ID No.4) sur la phase solide (protocole 1b), les échantillons testés (spécifiques de la capside) ne sont pas détectés, alors qu'ils sont trouvés positifs avec le protocole 1 a et dans un test anticorps classique (cf. Tableau 3).

**Tableau 3 :**

| **Echantillon** | **Densité optique** | | **Interprétation** | | **Test Anticorps** |
|---|---|---|---|---|---|
| | **1a** | **1b** | **1a** | **1b** | |
| 16 | 0,942 | 0,092 | **Positif** | Négatif | **Positif** |
| 38 | 0,409 | 0,041 | **Positif** | Négatif | **Positif** |
| 49 | 3,246 | 0,080 | **Positif** | Négatif | **Positif** |
| KJ9-1102-0025 | 0,916 | 0,054 | **Positif** | Négatif | **Positif** |

L'invention permet donc de détecter chez des sujets contaminés par le VHC des échantillons positifs en anticorps anti-capside, et négatifs en antigène capsidique.

### Exemple 3 : Détection simultanée d'antigène capsidique et d'anticorps anti-VHC dans un échantillon (sérum ou plasma)

Deux panels d'échantillons de séroconversion PHV 907 et PHV 917 (c'est à dire deux séries de sérums ou plasmas, prélevés chez deux patients après infection par le VHC ou en cours de séroconversion) commercialisés par BBI (Boston Biomedica Company, USA) ont été testés suivant le protocole 1a (cf. Tableaux 4 et 5).

**Tableau 4 :**

| **Echantillon BBI** | **Jour de prélèvement** | **Densité optique** | **Interprétation** | **Test Anticorps** | **Test PCR** |
|---|---|---|---|---|---|
| 907-1 | 0 | 0,482 | **Positif** | Négatif | **Positif** |
| 907-2 | + 4 | 0,344 | **Positif** | Négatif | **Positif** |
| 907-3 | + 7 | 0,325 | **Positif** | Négatif | **Positif** |
| 907-4 | + 13 | 0,318 | **Positif** | Négatif | **Positif** |
| 907-5 | + 18 | 0,395 | **Positif** | Négatif | **Positif** |
| 907-6 | + 21 | 0,855 | **Positif** | **Positif** | **Positif** |
| 907-7 | + 164 | 2,991 | **Positif** | **Positif** | **Positif** |

**Tableau 5 :**

| **Echantillon BBI** | **Jour de prélèvement** | **Densité optique** | **Interprétation** | **Test Anticorps** | **Test PCR** |
|---|---|---|---|---|---|
| 917-1 | 0 | 0,045 | Négatif | Négatif | Négatif |
| 917-2 | 13 | 0,803 | **Positif** | Négatif | **Positif** |
| 917-3 | 20 | 0,324 | **Positif** | Négatif | **Positif** |
| 917-4 | 22 | 0,448 | **Positif** | Négatif | **Positif** |
| 917-5 | 85 | 2,652 | **Positif** | **Positif** | Négatif |
| 917-6 | 131 | 2,582 | **Positif** | **Positif** | Négatif |
| 917-7 | 135 | 2,720 | **Positif** | **Positif** | **Positif** |
| 917-8 | 138 | 2,736 | **Positif** | **Positif** | Négatif |
| 917-9 | 146 | 3,023 | **Positif** | **Positif** | Négatif |
| 917-10 | 152 | 3,033 | **Positif** | **Positif** | Négatif |

Dans les tableaux 4 et 5, on observe, de manière corrélée à la présence de l'ARN viral (test PCR), un signal positif en antigène avec le protocole 1a selon l'invention ; de la même manière, une réponse positive est obtenue sur des prélèvements de séroconversion, positifs en anticorps et/ou en antigène. Ceci montre bien que les 2 détections (antigène capsidique et anticorps anti-capside) peuvent fonctionner simultanément dans le même essai, et sans interférence.

De plus, l'invention décrite présente un intérêt tout particulier d'un point de vue diagnostique vis à vis de la PCR, car elle permet de détecter des échantillons négatifs en PCR et positifs en anticorps.

### Exemple 4: Comparaison des performances de différents peptides mutés sur la détection d'anticorps anti-capside

Des échantillons sériques ou plasmatiques, prélevés chez des patients contaminés par le VHC et rassemblés dans des panels internes et commerciaux, positifs en anticorps (capside) et négatifs en antigène (capside), ont été testés suivant les méthodes décrites dans les protocoles 1c et 1d (cf Tableaux 6 et 7).

**Tableau 6: protocole 1c**

| **Echantillon** | **SEQ ID No. 5** | | **SEQ ID No.11** | | **SEQ ID No.8** | |
|---|---|---|---|---|---|---|
| | **DO** | **Interp** | **DO** | **Interp** | **DO** | **Interp** |
| 16 (1/2)* | 0.434 | **Pos** | 0,534 | **Pos** | 0,413 | **Pos** |
| 21 (1/2)* | 0,520 | **Pos** | 0,568 | **Pos** | 0,481 | **Pos** |
| 49 (1/20)* | 0,405 | **Pos** | 0,538 | **Pos** | 0,384 | **Pos** |
| O7 | 0,312 | **Pos** | 0,340 | **Pos** | 0,285 | **Pos** |

| | | | | | | |
|---|---|---|---|---|---|---|
| * : dilution | | | | | | |

**Tableau 7 : protocole 1d**

| **Echantillon** | **SEQ ID No. 4** | | **SEQ ID No. 15** | | **SEQ ID No.14** | |
|---|---|---|---|---|---|---|
| | **DO** | **Interp** | **DO** | **Interp** | **DO** | **Interp** |
| 16 (1/2)* | 0.438 | **Pos** | 0,362 | **Pos** | 0,327 | **Pos** |
| 21 (1/4)* | 0,257 | **Pos** | 0,218 | Neg | 0,197 | Neg |
| 38 (1/2)* | 0,310 | **Pos** | 0,221 | **Pos** | 0,206 | Neg |
| 49 (1/40)* | 0,308 | **Pos** | 0,228 | **Pos** | 0,201 | Neg |
| BCP 453 (1/4)* | 0,363 | **Pos** | 0,208 | Neg | 0,175 | Neg |
| KJ9-0025 (1/40)* | 0,263 | **Pos** | 0,237 | **Pos** | 0,212 | Neg |

| | | | | | | |
|---|---|---|---|---|---|---|
| * :dilution | | | | | | |

Les peptides mutés comparés dans le tableau 6 présentent des performances assez similaires sur les échantillons testés.

Par contre, les différences de réponse entre les peptides sont plus marquées dans le tableau 7. Les peptides SEQ ID No. 15 et SEQ ID No.14 ne permettent pas de détecter certains échantillons dilués anti-capside, les mêmes échantillons étant reconnus lorsque testés purs.

### Exemple 5 : Influence de la détection simultanée d'antigène capsidique et d'anticorps anti-VHC sur le diagnostic et la diminution de la fenêtre sérologique

Huit panels de séroconversions commercialisés (BBI, Boston Biomedica, US), (Impath, US) ont été testés avec le protocole selon l'invention (désigné test Combo) en suivant les protocoles 1a (avec le peptide C-G-G-Cap 1-75 (G34-G44-G47) contenant la SEQ ID No.4), 1c (avec le peptide C-G-G-Cap 1-75 (G31-G44-G47) contenant la SEQ ID No.5), et 1d (avec le peptide C-G-G-Cap 1-75 (G34-G44-G47) contenant la SEQ ID No.4).

Dans le tableau 8 sont reportés les résultats obtenus en détection d'antigène (test Elisa), ou d'ARN (PCR), et en détection d'anticorps et en Combo (formats 1a, 1c et 1 d) sur ces différentes séroconversions. Pour 4 séroconversions sur 8, les protocoles 1 a, 1c et 1d permettent de détecter le premier prélèvement positif de façon concomitante à l'apparition de l'ARN viral détecté par la PCR. Le nombre de jours moyens, entre la détection d'antigène et la détection d'anticorps correspondant à la réduction de la fenêtre sérologique, est de 33 jours pour les deux protocoles 1a, 1 c et 1d.

**Tableau 8 :**

| **Panel IMPATH/BBI** | Retard (jours) par rapport au 1er jour de détection ARN VHC | | | | | **Réduction de la fenêtre sérologique avec le test Combo Jours** |
|---|---|---|---|---|---|---|
| | **Ag VHC** | **Ac VHC** | **Combo VHC 1a** | **Combo VHC 1c** | **Combo VHC 1d** | |
| 6211 (NS3) | 0 | **46** | **10** | **10** | **NT** | **36** |
| 6225 (NS3) | 0 | **33** | **2** | **2** | **2** | **31** |
| 6227 (CAP-NS3-NS4) | 0 | **32** | **4** | **4** | **4** | **28** |
| 6229 (NS3) | 0 | **24** | **0** | **0** | **0** | **24** |
| 9041 (NS3) | 0 | **38** | **3** | **3** | **3** | **35** |
| 907 (CAP) | 0 | **13** | **0** | **0** | **0** | **13** |
| 6222 (CAP-NS3) | 0 | **23** | **0** | **0** | **0** | **23** |
| 917 (CAP-NS3) | 0 | **72** | **0** | **0** | **0** | **72** |
| **Moyenne = 33 jours** | | | | | | |

| | | | | | | |
|---|---|---|---|---|---|---|
| **NT = non testé** | | | | | | |

### Exemple 6 : Comparaison de performances et classement de diverses techniques sur des sérums de séroconversion

21 panels de séroconversion commercialisés (BBI, Boston Biomedica Company, USA ; Impath, USA), présentant des spécificités différentes ont été testés avec le test selon l'invention en suivant les protocoles 1a et 1c, et avec une série de tests commercialisés, qui sont en fait parmi les meilleurs selon le classement réalisé par la MDA (Medical Devices Agency) aux Etats-Unis en février 2002. Cette comparaison tient compte du nombre de jours de retard de détection par rapport à la détection d'ARN (test PCR). Ainsi la trousse présentant la plus petite moyenne générale est la plus performante en précocité de détection (tableau 9).

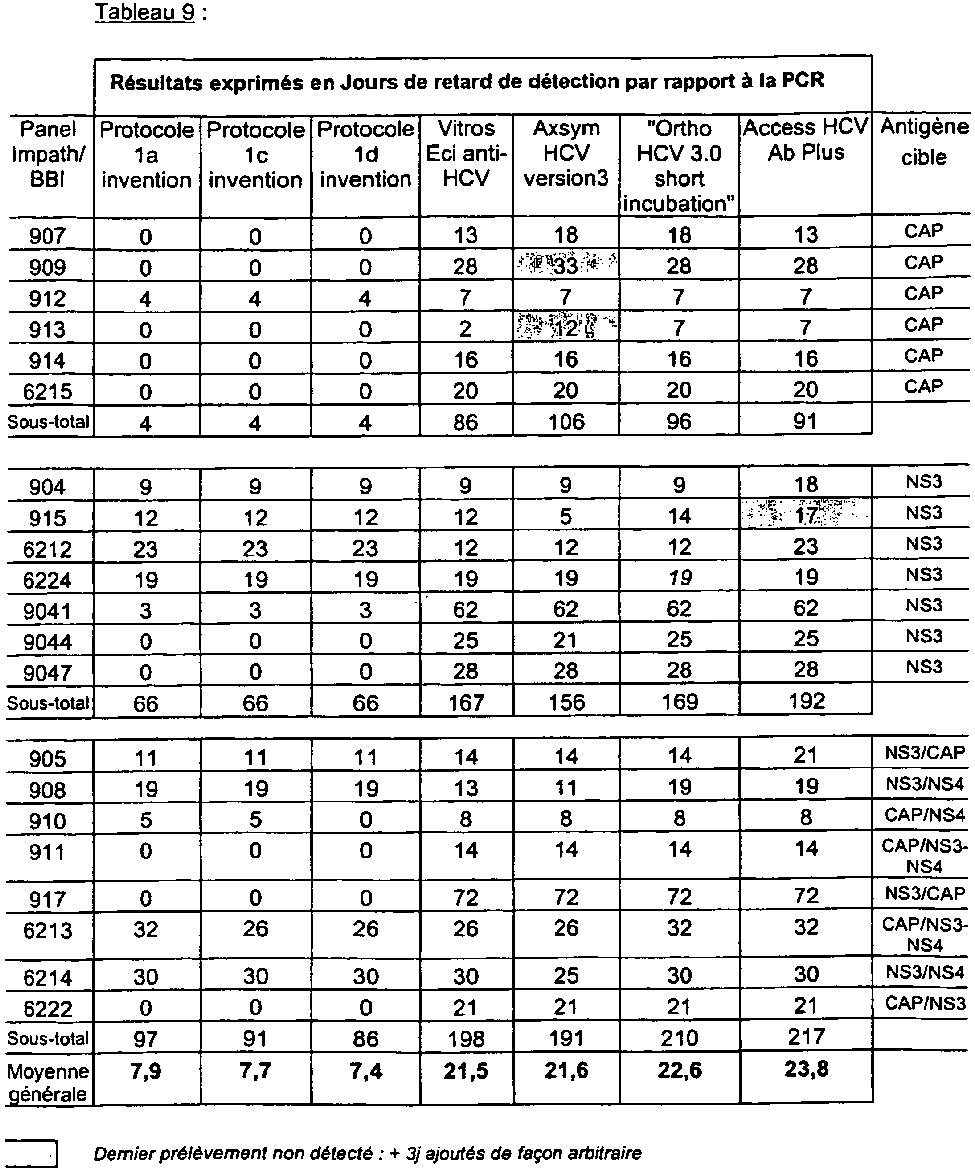

Les résultats générés avec les protocoles 1 a, 1c et 1d selon l'invention permettent une détection plus précoce de l'infection VHC (seulement 8 jours de retard en moyenne par rapport à la PCR pour le protocole 1a, 7,7 jours pour le protocole 1c, 7,4 jours de retard pour le protocole 1d), avant l'apparition des anticorps.

Ainsi le test décrit selon l'invention obtient la première place dans ce classement et cela avec les trois protocoles (1a, 1c et 1d) qui font appel à des couples d'anticorps et des peptides mutés différents.

En conclusion, les résultats obtenus avec les protocoles 1a, 1c et 1d selon l'invention montrent bien que cette dernière permet effectivement de détecter l'infection par le VHC très précocement, avec une sensibilité assez proche de celle de la PCR, tout en permettant de suivre l'évolution sérologique du patient après la séroconversion.

### Exemple 7 : Etude de spécificité sur les 3 protocoles Combo 1a, 1c et 1d

L'étude de spécificité a été réalisée sur des sérums de donneurs de sang (provenant de l'Etablissement Français du Sang, Rungis, France). Les résultats obtenus pour les différents protocoles 1a, 1c et 1d ont été reportés respectivement dans les tableaux 10, 11 et 12.

**Tableau 10 : Protocole 1a**

| | |
|---|---|
| Nombre de sérums trouvés négatifs | 968 |
| Sérums réactifs confirmés | 1 |
| Sérums réactifs confirmés (%) | 99,9 |
| Moyenne des négatifs en Ratio ( DO échantillon/Valeur seuil) | 0,23 |
| Ecart-type | 0,077 |
| Nombre d'écart-types pour la Valeur Seuil | 10 |

**Tableau 11 : Protocole 1 c**

| | |
|---|---|
| Nombre de sérums trouvés négatifs | 2475 |
| Sérums réactifs confirmés | 6 |
| Sérums réactifs confirmés (%) | 99,8 |
| Moyenne des négatifs en Ratio ( DO échantillon/Valeur seuil) | 0,14 |
| Ecart-type | 0,063 |
| Nombre d'écart-types pour la Valeur Seuil | 13,9 |

**Tableau 12 : Protocole 1d**

| | |
|---|---|
| Nombre de sérums trouvés négatifs | 2475 |
| Sérums réactifs confirmées | 4 |
| Sérums réactifs confirmés (%) | 99,8 |
| Moyenne des négatifs en Ratio ( DO échantiillon/Valeur seuil) | 0,18 |
| Ecart-type | 0,055 |
| Nombre d'écart-types pour la Valeur Seuil | 14,8 |

On note de bonnes performances en spécificité, quel que soit le protocole (1 a, 1 c ou 1d ) utilisé.

Comme on aura pu le constater tout au long de cette description, le test Combo VHC selon l'invention est capable, de façon surprenante, grâce à la capture de l'antigène de la capside et à celle des anticorps anti-capside sur une seule et même zone protéique de la capside, de fournir une sensibilité se rapprochant celle de la PCR et meilleure que celle de certaines techniques de l'art antérieur.

Ce résultat est surprenant, car en modifiant les peptides capside cibles, les inventeurs prenaient le risque de perdre la détection d'un certain nombre significatif d'anticorps.

### Exemple 8 : Détection d'une infection par le virus de l'Immunodéficience Humaine (VIH-1)

Le procédé décrit dans la présente invention s'applique également à la détection de l'infection due à toutes sortes de microorganismes infectieux (comme des virus, tels que, par exemple, les virus responsables des diverses hépatites, les rétrovirus, en particulier les rétrovirus responsables du SIDA chez l'homme (VIH-1, VIH-1 groupe O, VIH-2) ou le singe, le cytomégalovirus (CMV), les flavivirus, dont les virus de la dengue, ainsi que les bactéries, les parasites microbiens, etc.) pour lesquels une détection simultanée d'antigènes et d'anticorps est souhaitable.

L'exemple 8 montre l'application de la présente invention à la détection simultanée de l'antigène viral P25 (gag) du Virus de l'Immunodéficience Humaine (VIH-1, i.e. « HIV-1 » en langue anglaise) et d'anticorps anti-P25.

### Matériels communs pour les protocoles a, b, c:

**1)** Phase solide choisie: microplaque Maxisorp, Nunc (Danemark).
**2)** Diluant de la 1^{ère} étape : Tampon Tris, NaCl 0,05M, à pH 6,7 additionné de NP40 (IGEPAL CA 630, Sigma) à 0,25%.
**3)** Diluant de la 2^{éme} étape: tampon Tris NaCl 0,01 M, pH 7,4 additionné de Tween 20 à 0,1%
**4)** Solution de révélation : la solution de révélation était composée
   **4a)** d'un tampon substrat : Solution d'acide citrique (0,075M), et d'acétate de sodium (0,1M), à pH 4,0 , contenant de l'H₂O₂ à 0,015% et du Diméthylsulfoxyde (DMSO) (PROLABO) à 4%, et
   **4b)** d'un réactif chromogène : tetraméthylbenzidine (TMB, Sigma) à 0,7mM en concentration finale dans le tampon substrat.
**5)** Solution d'arrêt : H₂SO₄ 1 N.
   Matériels du protocole a : détection d'anticorps anti-P25 VIH1 Peptide synthétique muté correspondant à la séquence suivante et comportant les mutations (G295-G298-G310-G312-F316) par rapport à la séquence consensus de la protéine P25 du VIH-1 M soit

Conjugué anticorps polyclonal de mouton anti-IgG (H+L) humaines marqué à la peroxydase (Bio-Rad). Ce conjugué est dilué, avant emploi, dans le diluant de la 2^{éme} étape (décrit ci-dessus) de sorte à permettre d'obtenir *in fine,* avec un échantillon positif bien documenté, une densité optique élevée et ce, dans des conditions bien connues de l'homme du métier.

### Matériels pour le protocole b: détection d'antigène P25 VIH-1

Anticorps monoclonal anti-P25 Pm25 reconnaissant la séquence de la protéine P25 VIH-1 M suivante
₃₀₈QASQEVKNWMTETLL₃₂₂ (SEQ ID No.24)

Conjugué anticorps monoclonal Pm25 décrit ci-dessus marqué à la peroxydase. Ce conjugué est dilué, avant emploi, dans le diluant de la 2^{ème} étape (décrit ci-dessus) de sorte à permettre d'obtenir *in fine,* avec un échantillon positif bien documenté, une densité optique élevée et ce, dans des conditions bien connues de l'homme du métier.

### Matériels pour le protocole c: détection simultanée (« Combo ») d'anticorps anti-P25 et d'antigène P25 VIH-1

Peptide synthétique muté correspondant à la séquence suivante

Anticorps monoclonal Pm25 reconnaissant la séquence de la protéine P25 VIH-1 M suivante
₃₀₈QASQEVKNWMTETLL₃₂₂ (SEQ ID No.24)

Conjugué anticorps polyclonal de mouton anti-IgG (H+L) humaines marqué à la peroxydase (Bio-Rad).

Conjugué anticorps monoclonal Pm25 décrit ci-dessus marqué à la peroxydase.

Les 2 conjugués immuno-peroxydasiques ci-dessus sont dilués ensemble, avant emploi, dans le diluant de la 2^{ème} étape (décrit ci-dessus) de sorte à permettre d'obtenir *in fine,* avec un échantillon positif bien documenté, une densité optique élevée et ce, dans des conditions bien connues de l'homme du métier.

### Méthodes :

### Protocole a : détection des anticorps anti-P25 VIH-1

Le principe du test est basé sur une méthode immunoenzymatique de type indirect pour la détection des anticorps anti-P25 VIH-1.

II repose sur les étapes suivantes :
Une solution de sensibilisation est tout d'abord réalisée avec le peptide muté SEQ ID No.22 (G295-G298-G310-G312-F316) comprenant la séquence SEQ ID No.22 en tampon Carbonate pH 9,6

Les cupules d'une plaque de microtitrage (Nunc, Maxisorp) sont alors sensibilisées avec la solution ci-dessus à raison de 110 µl par cupule.

Les plaques de microtitrage sont mises à incuber pendant une nuit à température ambiante (18-24°C).

Après élimination de la solution de sensibilisation, les plaques sont lavées à l'aide d'un tampon phosphate (0,01 M, pH 7,4) contenant 0,1% de Tween 20, puis saturées par addition d'un tampon phosphate (0,01 M, pH 7) contenant 5% de saccharose, 25% de lait écrémé (Candia^{™}, France, ou tout autre lait écrémé équivalent du commerce).

Dans chaque cupule, on distribue successivement 80 µl de diluant de 1 ère étape, puis 20 µl d'échantillon (sérum ou plasma).

Le milieu réactionnel est mis à incuber à 37°C pendant 1 heure. Si des anticorps anti-P25 VIH-1 sont présents, ils se lient au peptide fixé sur la phase solide.

Les plaques sont ensuite lavées (3 fois) à l'aide d'une solution de lavage (tampon Tris NaCl 0,01 M, pH 7,4 additionné de Tween 20 à 0,1%).

Dans chaque cupule, on distribue 100 µl de diluant de 2ème étape, contenant les anticorps anti-IgG (H+L) humaines marqués à la peroxydase. Le milieu réactionnel est mis à incuber à température ambiante (18-24°C) pendant 30 minutes. Les anticorps anti-IgG (H+L) humaines marqués se fixent à leur tour aux anticorps spécifiques retenus sur la phase solide.

Les plaques sont ensuite lavées (5 fois) à l'aide d'une solution de lavage (tampon Tris NaCl 0,01M, pH 7,4 additionné de Tween 20 à 0,1%). Le conjugué anti-IgG humaines non lié est ainsi éliminé.

Dans chaque cupule, on distribue 100 µl de la solution de révélation. On laisse la réaction se développer à l'obscurité pendant 30 minutes à température ambiante (18-24°C).

On distribue ensuite 100 µl de solution d'arrêt dans chaque cupule.

Après arrêt de la réaction, la lecture de la densité optique est effectuée au spectrophotomètre à 450/620 nm.

### Protocole b : détection de l'antigène P25 VIH-1

Le principe du test est basé sur une méthode immunoenzymatique de type sandwich pour la détection d'antigène P25 VIH-1. Il repose sur les étapes suivantes :

Une solution de sensibilisation est tout d'abord réalisée avec l'anticorps monoclonal anti-P25 VIH-1 (Pm25) en tampon Carbonate pH 9,6.

Les cupules d'une plaque de microtitrage (Nunc, Maxisorp) sont alors sensibilisées avec la solution ci-dessus à raison de 110 µl par cupule.

Les étapes suivantes sont identiques à celles du protocole a jusqu'à la deuxième étape.

Pour la deuxième étape, on distribue 100 µl de diluant de 2^{ème} étape, contenant l'anticorps monoclonal Pm25 marqué à la peroxydase. Le milieu réactionnel est mis à incuber à température ambiante (18-24°C) pendant 30 minutes. L'anticorps monoclonal Pm25 marqué se fixe alors à l'antigène P25 retenu sur la phase solide, lorsqu'il est présent.

Les étapes finales sont identiques à celles du protocole a.

### Protocole c : détection simultanée (« Combo ») de l'antigène P25 VIH-1 et d'anticorps anti-P25 VIH-1 dans un échantillon (sérum ou plasma)

Le principe du test est basé sur une méthode immunoenzymatique de type sandwich pour la détection d'antigène et de type indirect pour la détection d'anticorps.

Il repose sur les étapes suivantes :
Une solution de sensibilisation est tout d'abord réalisée:
   - avec le peptide muté SEQ ID No.22 (G295-G298-G310-G312-F316) et,
   - avec l'anticorps monoclonal anti-P25 (Pm25) en tampon Carbonate pH 9,6.
Les cupules d'une plaque de microtitrage (Nunc, Maxisorp) sont alors sensibilisées avec la solution ci-dessus à raison de 110 µl par cupule.

Les plaques de microtitrage sont mises à incuber pendant une nuit à température ambiante (18-24°C).

Après élimination de la solution de sensibilisation, les plaques sont lavées à l'aide d'un tampon phosphate (0,01 M, pH 7,4) contenant 0,1% de Tween 20, puis saturées par addition d'un tampon phosphate (0,01M, pH 7) contenant 5% de saccharose, 25% de lait écrémé (Candia™, France, ou tout autre lait écrémé équivalent du commerce).

Dans chaque cupule, on distribue successivement 80 µl de diluant de 1^{ère} étape, puis 20 µl d'échantillon (sérum ou plasma).

Le milieu réactionnel est mis à incuber à 37°C pendant 1 heure. L'antigène P25 éventuellement présent se lie à l'anticorps monoclonal de la phase solide. De même, si des anticorps anti-P25 sont présents, ils se lient au peptide antigénique fixé sur la phase solide.

Les étapes suivantes sont identiques à celles du protocole 1a jusqu'à la deuxième étape.

Pour la deuxième étape, on distribue 100 µl de diluant de 2^{éme} étape, contenant l'anticorps monoclonal Pm25 marqué à la peroxydase et les anticorps anti-IgG (H+L) humaines marqués à la peroxydase. Le milieu réactionnel est mis à incuber à température ambiante (18-24°C) pendant 30 minutes. L'anticorps monoclonal Pm25 marqué se fixe alors à l'antigène P25 retenu sur la phase solide. De même, les anticorps anti-IgG (H+L) humaines marqués se fixent à leur tour aux anticorps spécifiques retenus sur la phase solide.

Les étapes finales sont identiques à celles du protocole a.

**Résultats :**

| Echantilion | Protocole a (D.O.) | Protocole b (D.O.) | Protocole c (D.O.) |
|---|---|---|---|
| **Positif en Anticorps anti-P25** | | | |
| Ech.1 | 0,138 | - | 0,316 |
| Ech.2 | 0,513 | - | 0,946 |
| **Positif en Antigène P25** | | | |
| N°1 | - | 1,104 | 1,151 |
| N°2 | - | 0,587 | 0,695 |
| **Négatif en Anticorps anti-P25 et en Antigène P25** | | | |
| Ech. 3 | 0,045 | 0,051 | 0,125 |
| Ech. 4 | 0,025 | 0,049 | 0,179 |

Comme on peut le constater pareillement ici, aussi, la capture de l'antigène P25 du VIH-1 et celle des anticorps anti-P25 du VIH-1 se font, dans la présente invention, sur une seule et même zone protéique de la capside : la zone antigénique capturant les anticorps anti-P25 (AA293-350) inclut bien la zone de spécificité antigénique (AA 308-322) par laquelle l'anticorps anti-P25 immobilisé (Pm25) capture l'antigène P25.

Il va de soi que l'homme du métier, en s'inspirant du présent exemple 8, par exemple, pourra aussi réaliser aisément un immunoessai de détection simultanée de l'antigène P25 (gag) du VIH-1, des anticorps anti-P25 du VIH-1 et des anticorps dirigés contre la glycoprotéine gp41 (enveloppe) du VIH-1. Pour la détection des anticorps anti-gp41 du VIH-1, il pourra utiliser, à titre d'exemple non exhaustif, soit la gp 41 entière, naturelle ou recombinée, en soi connue, soit un peptide contenant l'épitope immunodominant de la gp41, tel que celui décrit par la publication Gnann, JW., et al. (1987).

Font partie aussi de l'invention des immunoessais de détection simultanée de l'antigène P26 (gag) du VIH-2 (i.e. « HIV-2 » en langue anglaise), des anticorps anti-P26 du VIH-2 et des anticorps dirigés contre la glycoprotéine gp36 (enveloppe) du VIH-2. L'homme du métier pourra avoir recours, pour détecter l'antigène P26, à des anticorps anti-P26, en soi connus, et, pour détecter les anticorps anti-P26, à des peptides antigéniques de la p26, par exemple, dérivables de la séquence du VIH-2 publiée par Guyader et al. (1987). Pour la détection des anticorps anti-gp36, l'homme du métier pourra avoir recours, à titre d'exemple non exhaustif, soit à la gp 36 entière, naturelle ou recombinée, en soi connue, soit à un peptide contenant l'épitope immunodominant de la gp36, tel que celui décrit par la publication Gnann, JW., et al. (1987).

Il lui sera aussi possible de réaliser aisément un immunoessai « Combo VIH-1 + VIH-2 » alliant simultanément la détection de l'antigène P25 du VIH-1, de l'antigène P26 du VIH-2, des anticorps anti-P25 du VIH-1, des anticorps anti-P26 du VIH-2, des anticorps anti gp41 du VIH-1 et des anticorps anti-gp36 du VIH-2, qui fait aussi partie de l'invention.

### REFERENCES BIBLIOGRAPHIQUES

- Alcon et al., J. of Clin. Microbiol., 2002, vol. 40(2), pp. 376-381
- Atherton et al. (1989) "solid phase peptide synthesis, a practical approach", IRL Press, , Oxford University Press, pp. 25-34
- Benoit et al, (1982) PNAS USA, 79, pp. 917-921
- Berck et Schultz, (1986), Arch. Pathol. Lab. Med., 10, pp. 13-20
- Bukh, Semin. Liver Dis. (1995) 15 : 41-63 ;
- Cerino et al., (1991), J. Immunology, Vol. 147(8), pp. 2692-2696.
- Clayes et al. (1995), J of Medical Virology, 45, pp. 273-281
- Garson et al., (1990) Lancet, 336, pp. 878-879;
- Gnann, JW., et al. (1987) Science, 237 : pp. 1346-1349
- Guyader et al. (1987) Nature 326, pp. 662-669
- Hajime Tokita et al. (2000) J. Clin. Microbiol, Vol. 38, pp. 3450-3452
- Harlow et al. (1988) ed., "Antibodies : a laboratory manual"
- Hermanson Greg T., (1996) Bioconjugate techniques, Academic Press, New York, pp. 373-380 et pp. 580-583.
- Hosein et al. (1991) PNAS Vol. 88, May, pp. 3647 - 3651
- Icardi et al. (2001) J. Clin. Microbiol., 39, pp. 3110-3114
- Ishida, (1993), J. Clin. Microbiol., Vol. 31, No. 4, , pp. 936-940.
- Köhler et Milstein, (1975) Nature, 256, pp. 495-497
- Leahy et al. (1991) Third International Symposium on HCV, Strasbourg, poster B35
- Maiolini et al., (1978) Journal of Immunological Methods, 20, pp. 25-34
- Marks et al. (1991) J. Mol. Biol, 222, pp. 581-597
- Merrifield (1963) J. Amer. Chem. Soc,, 85, pp. 2149-2154 ;
- Nasoff et al. (1991) PNAS Vol. 88, No. 12, pp. 5462 - 5466,
- Okamoto et al. (1990a) Japan J. Exp. Med., vol. 60(3), pp. 167-177
- Okamoto et al. (1990b)Japan J. Exp. Med., vol. 60(4), pp. 223-233
- Peterson et al. (2000) Vox sanguinis, Vol. 78, pp. 80-85,
- Ratner et al. (1985) Nature, 313, pp. 277-284
- Säliberg et al. (1992) J. Clin. Microbiology, Vol. 30(8), pp. 1989-1994
- Sanchez-Pescador et al. (1985) Science, 227, pp.484-492
- Scott et al. (1990),Science, 249, pp. 386-390
- Sheppard, dans « Peptides 1971 », Nesvadba H (ed.) North Holland, Amsterdam, p. 111
- Shieh et al. (1991) Laboratory Investigation Vol. 65, No. 4, pp. 408 - 411
- Simmonds, (1995), Hepatology,, 21, pp. 570-583
- Stuyver et al. (1994), P.N.A.S. USA, 91, pp. 10134-10138
- Takahashi et al. (1992) J. Gen. Virol., Vol. 73(3), , London, pp. 667-672
- Wain-Hobson et al. (1985) Cell, 40, pp. 9-17
- Yang et al. (1995) Clin. Exp Immunol., 101, pp. 272-277
- Yang et al. (1999), J of Medical Virology, 57, pp. 345-350

## Revendications

1. Procédé de détection *in vitro* d'une infection par un microorganisme dans un échantillon biologique, comprenant la détection simultanée d'au moins un antigène dudit microorganisme et d'un anticorps dirigé contre ledit microorganisme, présents dans l'échantillon biologique, procédé comprenant
a) la mise en présence de l'échantillon biologique avec un anticorps de capture dirigé contre ledit microorganisme immobilisé sur une phase solide, et un antigène de capture dérivé dudit microorganisme immobilisé sur une phase solide;
b) l'incubation du mélange dans des conditions permettant la formation de complexes antigènes-anticorps ;
c) la révélation des complexes antigènes-anticorps formés, qui met en oeuvre un anticorps de détection, marqué, capable de se lier à l'antigène dudit microorganisme capturé et/ou éventuellement aussi un antigène de détection, marqué, capable de se lier à l'anticorps dirigé contre ledit microorganisme capturé;
et dans lequel l'antigène de capture et/ou l'antigène de détection, marqué, dudit microorganisme comprend un fragment antigénique dudit microorganisme dont au moins un épitope est dètruit, étant rendu incapable d'être reconnu par l'anticorps de capture et/ou de détection dirigé contre l'antigène tout en étant capable de lier les anticorps dirigés contre le microorganisme, par reconnaissance d'autres sites épitopiques restés intacts;
et l'anticorps de capture et/ou de détection, qui sont des anticorps monoclonaux ou polyclonaux monospécfiques, reconnaît ledit au moins un épitope, intact, de l'antigène capturé.

2. Procédé selon la revendication 1, dans lequel le microorganisme est choisi dans le groupe constitué par les virus, les bactéries et les parasites microbiens.

3. Procédé selon la revendication 2, dans lequel le microorganisme est un virus de l'hépatite C (VHC).

4. Procédé selon la revendication 3, dans lequel l'antigène de capture comprend un fragment antigénique de la protéine de capside du VHC, dans lequel au moins un site épitopique est détruit.

5. Procédé selon la revendication 3 ou 4, dans lequel l'antigène de capture comprend un fragment antigénique de la protéine non structurale du VHC NS3 ou NS4.

6. Procédé selon la revendication 2, dans lequel le microorganisme est un virus de l'immunodéficience humaine (VIH).

7. Procédé selon la revendication 6, dans lequel le VIH est un VIH-1 et/ou un VIH-2.

8. Procédé selon la revendication 6 ou 7, dans lequel l'antigène de capture comprend un fragment antigénique de la protéine gag du VIH, dans lequel au moins un site épitopique est détruit.

9. Procédé selon la revendication 6 ou 7, dans lequel l'antigène de capture comprend un fragment antigénique d'une protéine d'enveloppe du VIH.

10. Procédé selon l'une quelconque des revendications 3 à 9, dans lequel l'antigène de capture contient au moins deux sites épitopiques différents détruits.

11. Procédé selon l'une quelconque des revendications 3 à 10, dans lequel l'antigène de capture contient un site épitopique détruit par substitution, délétion, ou insertion, d'au moins un acide aminé.

12. Procédé selon l'une quelconque des revendications 3 à 11, dans lequel l'anticorps de détection est ajouté au mélange après que les complexes antigènes-anticorps se sont formés.

13. Procédé selon l'une quelconque des revendications 3 à 12, dans lequel l'anticorps de détection, et/ou l'antigène de détection, est mis en présence de l'échantillon biologique en même temps que l'anticorps de capture et l'antigène de capture.

14. Procédé selon la revendication 3, comprenant
a) la mise en présence de l'échantillon biologique avec un anticorps de capture du VHC et un antigène de capture du VHC fixés sur une phase solide;
b) l'incubation du mélange dans des conditions permettant la formation de complexes antigènes-anticorps ;
c) la séparation de la phase solide et de la phase liquide ;
d) la mise en contact de la phase solide avec, d'une part, un anticorps de détection, marqué, capable de lier l'antigène de VHC capturé, et d'autre part, un anticorps, anti-immunoglobuline ou anti-isotype, ou un antigène de détection, marqué, capable de lier l'anticorps anti-VHC capturé,
l'antigène de capture, et l'antigène de détection, d'anticorps anti-VHC comprenant des fragments antigéniques de la protéine de capside du VHC, dont deux épitopes ont été détruits,
et les anticorps de capture et de détection reconnaissant chacun un desdits épitopes, intacts, de l'antigène de capside capturé.

15. Procédé selon la revendication 14, où l'anticorps de capture et l'anticorps de détection reconnaissent chacun un épitope sélectionné dans le groupe d'épitopes de séquences suivantes :
⁴⁴LGVR⁴⁷ (SEQ ID No.19), ³⁰IVGGVYL³⁶ (SEQ ID No.20) et ²⁹QIVGGV³⁴ (SEQ ID No.21).

16. Procédé de détection selon la revendication 6 ou 7, comprenant:
a) la mise en présence de l'échantillon avec un anticorps de capture du VIH et un antigène de capture du VIH fixés sur une phase solide;
b) l'incubation du mélange dans des conditions permettant la formation de complexes antigènes-anticorps ;
c) la séparation de la phase solide et de la phase liquide ;
d) la mise en contact de la phase solide avec un anticorps de détection, marqué, capable de lier l'antigène de VIH capturé, et un ou des anticorps anti-immunoglobuline, ou anti-isotype, marqué, capable de lier l'anticorps anti-VIH capturé,
l'antigène de capture d'anticorps anti-VIH comprenant un fragment antigénique de la protéine gag du VIH, dont au moins un épitope a été détruit,
et les anticorps de capture et de détection reconnaissant chacun un desdits épitopes, intacts, de l'antigène de capside capturé.

17. Procédé selon la revendication 16, où l'anticorps de capture et l'anticorps de détection reconnaissent chacun un épitope de séquence QASQEVKNWMTETLL (SEQ ID No.24).

18. Procédé selon l'une quelconque des revendications 14 à 17, où la mise en présence de l'échantillon biologique avec ledit anticorps de capture et ledit antigène de capture fixés sur une phase solide se réalise en présence d'au moins un détergent de type non-ionique.

19. Procédé selon la revendication 18, dans lequel ledit détergent non ionique est le NP40.

20. Trousse utile pour la détection d'une infection par un virus de l'hépatite C (VHC) ou un virus de l'immunodéficience humaine (VIH) dans un échantillon biologique, comprenant :
- un antigène de capture, immobilisé sur une phase solide, qui comprend un fragment antigénique d'une protéine dudit VHC ou VIH, ledit fragment comprenant au moins un épitope détruit, étant rendu incapable d'être reconnu par un anticorps de capture et un anticorps de détection dirigés contre ledit au moins un épitope, intact, sélectionné dans le groupe d'épitopes de séquences suivantes : ⁴⁴LGVR⁴⁷ (SEQ ID No.19), ³⁰IVGGVYL³⁶ (SEQ ID No.20), ²⁹QIVGGV³⁴ (SEQ ID No.21), QASQEVKNWMTETLL (SEQ ID No.24) et QTDPAVKNWMTQTLL (SEQ ID No.25), tout en conservant la capacité de lier les anticorps dirigés contre le microorganisme éventuellement présents dans l'échantillon biologique, par reconnaissance d'autres sites épitopiques restés intacts;
- ledit anticorps de détection, monoclonal ou polyclonal monospécifique, dirigé contre ledit au moins un épitope, intact, de ladite protéine du VHC ou VIH; et
- ledit anticorps de capture monoclonal ou polyclonal monospécfique, immobilisé sur une phase solide, dirigé contre ledit au moins un épitope, intact, de ladite protéine du VHC ou VIH;
ladite protéine du VHC étant la protéine de capside du VHC et l'anticorps de capture et/ou de détection reconnaissant un épitope sélectionné dans le groupe d'épitopes de séquences suivantes : ⁴⁴LGVR⁴⁷ (SEQ ID No.19), ³⁰IVGGVYL³⁶ (SEQ ID No.20) et ²⁹QIVGGV³⁴ (SEQ ID No,21), ou
ladite protéine du VIH étant la protéine P25 du VIH-1 et l'anticorps de capture et/ou de détection reconnaissant l'épitope de séquence QASQEVKNWMTETLL (SEQ ID No.24), ou
ladite protéine du VIH étant la protéine P26 du VIH-2 et l'anticorps de capture et/ou de détection reconnaissant l'épitope de séquence QTDPAVKNWMTQTLL (SEQ ID No.25).

21. Trousse selon la revendication 20, dans laquelle lesdits anticorps de capture et de détection sont dirigés contre un épitope identique, intact, de ladite protéine du VHC ou VIH.

22. Trousse selon la revendication 20, dans laquelle
- ledit antigène de capture comprend un fragment antigénique de ladite protéine dudit VHC ou VIH, ledit fragment comprenant au moins deux épitopes détruits étant rendu incapable d'être reconnu par ledit anticorps de capture et ledit anticorps de détection et
- ledit anticorps de capture est dirigé contre l'un desdits deux épitopes, intacts, de ladite protéine du VHC ou VIH, tandis que l'anticorps de détection est dirigé contre l'autre desdits deux épitopes, intacts, de ladite protéine du VHC ou VIH.

23. Trousse selon l'une des revendications 20 à 22, comprenant, outre l'antigène de capture, un moyen de détection desdits anticorps anti-VHC présents dans l'échantillon biologique et complexés audit antigène de capture.

24. Trousse selon l'une des revendications 20 à 22, comprenant, outre l'antigène de capture, un moyen de détection desdits anticorps anti-VIH présents dans l'échantillon biologique et complexés audit antigène de capture.

25. Trousse selon la revendication 23 ou 24 dans laquelle le moyen de détection est un anticorps anti-immunoglobuline ou anti-isotype marqué.

26. Trousse selon l'une des revendications 20 à 23, comprenant :
a) un antigène de capture qui comprend un fragment antigénique d'une protéine du VHC, ledit fragment comprenant au moins deux épitopes détruits, tout en conservant la capacité de lier les anticorps anti-VHC éventuellement présents dans un échantillon biologique ;
b) un anticorps de capture dirigé contre l'un desdits épitopes, intacts, de ladite protéine du VHC; ledit antigène de capture et ledit anticorps de capture étant immobilisés sur une phase solide ; et
c1) un anticorps de détection, marqué, dirigé contre un autre desdits épitopes, intacts, de ladite protéine du VHC, et/ou
c2) éventuellement un antigène de détection, marqué, qui est und fragment antigénique d'une protéine du VHC, ledit fragment comprenant au moins un épitope détruit, tout en conservant la capacité de lier les anticorps anti-VHC éventuellement présents dans un échantillon biologique.

27. Trousse selon la revendication 25 comprenant :
a) l'antigène de capture, qui comprend un fragment antigénique d'une protéine d'un VIH, ledit fragment comprenant au moins un épitope détruit, tout en conservant la capacité de lier les anticorps anti-VIH éventuellement présents dans un échantillon biologique ;
b) un anticorps de capture dirigé contre l'un desdits épitopes, intacts, de ladite protéine du VIH ;
ledit antigène de capture et ledit anticorps de capture étant immobilisés sur une phase solide ;
c1) un anticorps de détection, marqué, dirigé contre un autre desdits épitopes, intacts, de ladite protéine du VIH,
c2) et/ou éventuellement un antigène de détection, marqué, qui est un fragment antigénique d'une protéine du VIH, ledit fragment comprenant au moins un épitope détruit, tout en conservant la capacité de lier les anticorps anti-VIH éventuellement présents dans un échantillon biologique.

28. Trousse selon l'une des revendications 20 à 27, comprenant, en outre, au moins un détergent de type non-ionique.

29. Trousse selon la revendication 28, dans laquelle ledit détergent de type non-ionique est le NP40.

30. Peptide ou polypeptide issu de la protéine de capside du VHC, portant au moins un site épitopique intact et comprenant l'une quelconque des séquences SEQ ID No.4 à SEQ ID N.18.

31. Peptide ou polypeptide issu de la protéine gag d'un VIH, portant au moins un site épitopique intact et comprenant la séquence SEQ ID No.22.

## Patentansprüche

1. Verfahren zur in-vitro-Detektion einer Infektion durch einen Mikroorganismus in einer biologischen Probe, das die gleichzeitige Detektion wenigstens eines Antigens des Mikroorganismus und eines Antikörpers, der gegen den Mikroorganismus gerichtet ist, die in der biologischen Probe vorliegen, umfasst, wobei das Verfahren umfasst
a) Zusammenbringen der biologischen Probe mit einem Fangantikörper, der gegen den Mikroorganismus gerichtet ist, der auf einer festen Phase immobilisiert ist, und einem Fangantigen, das von dem Mikroorganismus stammt, das auf einer festen Phase immobilisiert ist;
b) Inkubation des Gemisches unter Bedingungen, die die Bildung von Antigen-Antikörper-Komplexen erlauben;
c) Nachweis der gebildeten Antigen-Antikörper-Komplexe unter Verwendung eines Detektionsantikörpers, der markiert ist, fähig ist, an das Antigen des Mikroorganismus, das gefangen wurde, zu binden, und/oder gegebenenfalls auch eines Detektionsantigens, das markiert ist, fähig ist, an den Antikörper, der gegen den Mikroorganismus gerichtet ist, der gefangen wurde, zu binden;
in dem das Fangantigen und/oder das Detektionsantigen, das markiert ist, des Mikroorganismus ein antigenes Fragment des Mikroorganismus umfasst, von dem wenigstens ein Epitop zerstört ist, wobei es unfähig gemacht wurde, durch den Fang- und/oder Detektionsantikörper, der gegen das Antigen gerichtet ist, erkannt zu werden, jedoch fähig ist, durch Erkennung anderer epitopischer Stellen, die intakt geblieben sind, die Antikörper zu binden, die gegen den Mikroorganismus gerichtet sind,
und in dem der Fang- und/oder Detektionsantikörper, die monoklonale oder polyklonale monospezifische Antikörper sind, das wenigstens eine intakte Epitop des gefangenen Antigens erkennt.

2. Verfahren gemäß Anspruch 1, wobei der Mikroorganismus aus der Gruppe, bestehend aus Viren, Bakterien und mikrobiellen Parasiten, ausgewählt ist.

3. Verfahren gemäß Anspruch 2, wobei der Mikroorganismus ein Hepatitis C-Virus (HCV) ist.

4. Verfahren gemäß Anspruch 3, wobei das Fangantigen ein antigenes Fragment des Capsidproteins des HCV, in dem wenigstens eine epitopische Stelle zerstört ist, umfasst.

5. Verfahren gemäß Anspruch 3 oder 4, wobei das Fangantigen ein antigenes Fragment des nicht-strukturellen Proteins des HCV NS3 oder NS4 umfasst.

6. Verfahren gemäß Anspruch 2, wobei der Mikroorganismus ein Virus der humanen Immundefizienz (HIV) ist.

7. Verfahren gemäß Anspruch 6, wobei das HIV ein HIV-1 und/oder ein HIV-2 ist.

8. Verfahren gemäß Anspruch 6 oder 7, wobei das Fangantigen ein antigenes Fragment des gag-Proteins des HIV, in dem wenigstens eine epitopische Stelle zerstört ist, umfasst.

9. Verfahren gemäß Anspruch 6 oder 7, wobei das Fangantigen ein antigenes Fragment eines Hüllproteins des HIV umfasst.

10. Verfahren gemäß einem der Ansprüche 3 bis 9, wobei das Fangantigen wenigstens zwei unterschiedliche zerstörte epitopische Stellen enthält.

11. Verfahren gemäß einem der Ansprüche 3 bis 10, wobei das Fangantigen eine epitopische Stelle enthält, die durch Substitution, Deletion oder Insertion wenigstens einer Aminosäure zerstört ist.

12. Verfahren gemäß einem der Ansprüche 3 bis 11, wobei der Detektionsantikörper dem Gemisch zugesetzt wird, nachdem sich die Antigen-Antikörper-Kompfexe gebildet haben.

13. Verfahren gemäß einem der Ansprüche 3 bis 12, wobei der Detektionsantikörper und/oder das Detektionsantigen zur selben Zeit wie der Fangantikörper und das Fangantigen mit der biologischen Probe in Kontakt gebracht werden.

14. Verfahren gemäß Anspruch 3, umfassend
a) Zusammenbringen der biologischen Probe mit einem Fangantikörper des HCV und einem Fangantigen des HCV, die auf einer festen Phase fixiert sind;
b) Inkubation des Gemisches unter Bedingungen, die die Bildung von Antigen-Antikörper-Komplexen erlauben;
c) Trennung der festen Phase von der flüssigen Phase;
d) In-Kontakt-Bringen der festen Phase mit einerseits einem Detektionsantikörper, der markiert ist, in der Lage ist, das Antigen des HCV, das gefangen wurde, zu binden, und andererseits einem Anti-Immunglobulin- oder Anti-Isotyp-Antikörper oder einem Detektionsantigen, das markiert ist, fähig ist, den Antikörper gegen HCV, der gefangen wurde, zu binden,
wobei das Fangantigen und das Detektionsantigen von Anti-HCV-Antikörpern antigene Fragmente des Capsidproteins des HCV, von denen zwei Epitope zerstört wurden, umfassen
und die Fang- und Detektionsantikörper jeweils eines der intakten Epitope des gefangenen Capsidantigens erkennen.

15. Verfahren gemäß Anspruch 14, wobei der Fangantikörper und der Detektionsantikörper jeweils ein Epitop erkennen, das aus der Gruppe von Epitopen der folgenden Sequenzen ausgewählt ist: ⁴⁴LGVR⁴⁷ (SEQ ID NO.19), ³⁰IVGGVYL³⁵ (SEQ ID NO.20) und ²⁹QIVGGV³⁴ (SEQ ID NO.21).

16. Verfahren zur Detektion gemäß Anspruch 6 oder 7, umfassend:
a) Zusammenbringen der Probe mit einem Fangantikörper des HIV und einem Fangantigen des HIV, die auf einer festen Phase fixiert sind;
b) Inkubation des Gemisches unter Bedingungen, die die Bildung von Antigen-Antikörper-Komplexen erlauben;
c) Trennung der festen Phase von der flüssigen Phase;
d) In-Kontakt-Bringen der festen Phase mit einem Detektionsantikörper, der markiert ist, fähig ist, das Antigen des HIV, das gefangen ist, zu binden, und einem Anti-Immunglobulin- oder Anti-Isotyp-Antikörper oder Anti-Immunglobulin- oder Anti-Isotyp-Antikörpern, der/die markiert ist/sind, fähig ist/sind, den gefangenen Anti-HIV-Antikörper zu binden,
wobei das Fangantigen von Anti-HIV-Antikörpern ein antigenes Fragment des gag-Proteins des HIV umfasst, bei dem wenigstens ein Epitop zerstört wurde,
und wobei die Fang- und Detektionsantikörper jeweils eines der genannten intakten Epitope des gefangenen Capsidantigens erkennen.

17. Verfahren gemäß Anspruch 16, wobei der Fangantikörper und der Detektionsantikörper jeweils ein Epitop der Sequenz QASQEVKNWMTETLL (SEQ ID NO.24) erkennen.

18. Verfahren gemäß einem der Ansprüche 14 bis 17, wobei das Zusammenbringen der biologischen Probe mit dem Fangantikörper und dem Fangantigen, die auf einer festen Phase fixiert sind, in Gegenwart wenigstens eines Detergens von nichtionischem Typ erfolgt.

19. Verfahren gemäß Anspruch 18, wobei das nicht-ionische Detergens NP40 ist.

20. Kit, der zur Detektion einer Infektion durch einen Hepatitis-C-Virus (HCV) oder einen Virus der humanen Immundefizienz (HIV) in einer biologischen Probe verwendbar ist, umfassend:
- ein Fangantigen, das auf einer festen Phase immobilisiert ist, das ein antigenes Fragment eines Proteins des HCV oder HIV umfasst, wobei das Fragment wenigstens ein zerstörtes Epitop umfasst, das unfähig gemacht wurde, durch einen Fangantikörper und einen Detektionsantikörper erkannt zu werden, die gegen das genannte wenigstens eine intakte Epitop gerichtet sind, das aus der Gruppe von Epitopen der folgenden Sequenzen ausgewählt ist: ⁴⁴LGVR⁴⁷ (SEQ ID NO.19), ³⁰IVGGVYL³⁶ (SEQ ID NO.20), ²⁹QIVGGV³⁴ (SEQ ID NO.21), QASQEVKNWMTETLL (SEQ ID NO.24) und QTDPAVKNWMTQTLL (SEQ ID NO.25), das jedoch die Fähigkeit, die Antikörper, die gegen den Mikroorganismus gerichtet sind, die gegebenenfalls in der biologischen Probe vorliegen, durch Erkennung anderer epitopischer Stellen, die intakt geblieben sind, zu binden, konserviert hat;
- den genannten monoklonalen oder polyklonalen monospezifischen Detektionsantikörper, der gegen das genannte wenigstens eine intakte Epitop des Proteins des HCV oder HIV gerichtet ist, und
- den monoklonalen oder polyklonalen monospezifischen Fangantikörper, der auf einer festen Phase immobilisiert ist, der gegen das wenigstens eine intakte Epitop des genannten Proteins des HCV oder HIV gerichtet ist;
wobei das genannte Protein des HCV das Capsidprotein des HCV ist, und der Fang- und/oder Detektionsantikörper ein Epitop erkennt, das ausgewählt ist aus der Gruppe von Epitopen der folgenden Sequenzen: ⁴⁴LGVR⁴⁷ (SEQ ID NO.19), ³⁰IVGGVYL³⁶ (SEQ ID NO.20) und ²⁹QIVGGV³⁴ (SEQ ID NO.21), oder
das genannte Protein des HIV das Protein P25 des HIV-1 ist, und der Fang- und/oder Detektionsantikörper das Epitop der Sequenz QASQEVKNWMTETLL (SEQ ID NO.24) erkennt, oder
das genannte Protein des HIV das Protein P26 des HIV-2 ist, und der Fang- und/oder Detektionsantikörper das Epitop der Sequenz QTDPAVKNWMTQTLL (SEQ ID NO.25) erkennt.

21. Kit gemäß Anspruch 20, in dem die Fang- und Detektionsantikörper gegen ein identisches intaktes Epitop des Proteins des HCV oder HIV gerichtet sind.

22. Kit gemäß Anspruch 20, in dem
- das Fangantigen ein antigenes Fragment des Proteins des HCV oder HIV umfasst, wobei das Fragment wenigstens zwei zerstörte Epitope umfasst, die unfähig gemacht wurden, durch den Fangantikörper und den Detektionsantikörper erkannt zu werden, und
- der Fangantikörper gegen eines der zwei intakten Epitope des Proteins des HCV oder HIV gerichtet ist, während der Detektionsantikörper gegen das andere der zwei intakten Epitope des Proteins des HCV oder HIV gerichtet ist.

23. Kit gemäß einem der Ansprüche 20 bis 22, der außer dem Fangantigen ein Mittel zur Detektion der Anti-HCV-Antikörper, die in der biologischen Probe vorliegen und mit dem Fangantigen komplexiert sind, umfasst.

24. Kit gemäß einem der Ansprüche 20 bis 22, der außer dem Fangantigen ein Mittel zur Detektion der Anti-HIV-Antikörper, die in der biologischen Probe vorliegen und mit dem Fangantigen komplexiert sind, umfasst.

25. Kit gemäß Anspruch 23 oder 24, in dem das Mittel zur Detektion ein markierter Anti-Immunglobulin- oder Anti-Isotyp-Antikörper ist.

26. Kit gemäß einem der Ansprüche 20 bis 23, umfassend:
a) ein Fangantigen, das ein antigenes Fragment eines Proteins des HCV umfasst, wobei das Fragment wenigstens zwei zerstörte Epitope umfasst, allerdings die Fähigkeit, die Anti-HCV-Antikörper, die gegebenenfalls in einer biologischen Probe vorliegen, zu binden, konserviert hat;
b) einem Fangantikörper, der gegen eines der intakten Epitope des Proteins des HCV gerichtet ist; wobei das Fangantigen und der Fangantikörper auf einer festen Phase immobilisiert sind, und
c1) einen markierten Detektionsantikörper, der gegen ein anderes der intakten Epitope des Proteins des HCV gerichtet ist, und/oder
c2) gegebenenfalls ein markiertes Detektionsantigen, das ein antigenes Fragment eines Proteins des HCV ist, wobei das Fragment wenigstens ein zerstörtes Epitop umfasst, jedoch die Fähigkeit, die Anti-HCV-Antikörper, die gegebenenfalls in einer biologischen Probe vorliegen, zu binden, konserviert hat.

27. Kit gemäß Anspruch 25, umfassend:
a) ein Fangantigen, das ein antigenes Fragment eines Proteins des HIV umfasst, wobei das Fragment wenigstens zwei zerstörte Epitope umfasst, allerdings die Fähigkeit, die Anti-HIV-Antikörper, die gegebenenfalls in einer biologischen Probe vorliegen, zu binden, konserviert hat;
b) einem Fangantikörper, der gegen eines der intakten Epitope des Proteins des HIV gerichtet ist;
wobei das Fangantigen und der Fangantikörper auf einer festen Phase immobilisiert sind, und
c1) einen markierten Detektionsantikörper, der gegen ein anderes der intakten Epitope des Proteins des HIV gerichtet ist,
c2) und/oder gegebenenfalls ein markiertes Detektionsantigen, das ein antigenes Fragment eines Proteins des HIV ist, wobei das Fragment wenigstens ein zerstörtes Epitop umfasst, jedoch die Fähigkeit, die Anti-HIV-Antikörper, die gegebenenfalls in einer biologischen Probe vorliegen, zu binden, konserviert hat.

28. Kit gemäß einem der Ansprüche 20 bis 27, der außerdem wenigstens ein Detergens des nicht-ionischen Typs umfasst.

29. Kit gemäß Anspruch 28, wobei das Detergens des nicht-ionischen Typs NP40 ist.

30. Peptid oder Polypeptid, das vom Capsidprotein des HCV stammt, das wenigstens eine intakte epitopische Stelle trägt und eine der Sequenzen SEQ ID NO.4 bis SEQ ID NO.18 umfasst.

31. Peptid oder Polypeptid, das vom gag-Protein eines HIV stammt, das wenigstens eine intakte epitopische Stelle trägt und die Sequenz SEQ ID NO.22 umfasst.

## Claims

1. Method for the *in vitro* detection of an infection by a micro-organism in a biological sample, comprising the simultaneous detection of at least one antigen of said micro-organism and an antibody directed against said micro-organism, which are present in the biological sample, a method comprising
a) bringing the biological sample together with a capture antibody directed against said micro-organism immobilized on a solid phase, and a capture antigen derived from said micro-organism immobilized on a solid phase;
b) incubating the mixture in conditions which allow the formation of antigen / antibody complexes;
c) revealing the antigen / antibody complexes formed, which uses a detection antibody, labelled, capable of being bound to the antigen of said captured micro-organism and/or optionally also a detection antigen, labelled, capable of being bound to the antibody directed against said captured micro-organism;
and in which the capture antigen and/or the detection antigen, labelled, of said micro-organism comprises an antigenic fragment of said micro-organism, at least one epitope of which is destroyed, being made incapable of being recognized by the capture antibody and/or the detection antibody directed against the antigen whilst being capable of binding the antibodies directed against the micro-organism, by recognition of other epitopic sites which remain intact;
and the capture antibody and/or the detection antibody, which are monospecific monoclonal or polyclonal antibodies, recognizes or recognize said at least one epitope, intact, of the captured antigen.

2. Method according to Claim 1, in which the micro-organism is selected from the group comprising viruses, bacteria and microbial parasites.

3. Method according to Claim 2, in which the micro-organism is a hepatitis C virus (HCV).

4. Method according to Claim 3, in which the capture antigen comprises an antigenic fragment of the capsid protein of the HCV, in which at least one epitopic site is destroyed.

5. Method according to Claim 3 or 4, in which the capture antigen comprises an antigenic fragment of the non-structural protein of the HCV NS3 or NS4.

6. Method according to Claim 2, in which the micro-organism is a human immunodeficiency virus (HIV).

7. Method according to Claim 6, in which the HIV is an HIV-1 and/or an HIV-2.

8. Method according to Claim 6 or 7, in which the capture antigen comprises an antigenic fragment of the gag protein of the HIV in which at least one epitopic site is destroyed.

9. Method according to Claim 6 or 7, in which the capture antigen comprises an antigenic fragment of an envelope protein of the HIV.

10. Method according to any one of Claims 3 to 9, in which the capture antigen contains at least two destroyed different epitopic sites.

11. Method according to any one of Claims 3 to 10, in which the capture antigen contains an epitopic site destroyed by the substitution, deletion or insertion of at least one amino acid.

12. Method according to any one of Claims 3 to 11, in which the detection antibody is added to the mixture after the antigen / antibody complexes have been formed.

13. Method according to any one of Claims 3 to 12, in which the detection antibody and/or the detection antigen is or are brought together with the biological sample at the same time as the capture antibody and the capture antigen.

14. Method according to Claim 3, comprising
a) bringing the biological sample together with a capture antibody of the HCV and a capture antigen of the HCV fixed on a solid phase;
b) incubating the mixture in conditions which allow the formation of antigen / antibody complexes;
c) separating the solid phase and the liquid phase;
d) bringing the solid phase into contact firstly with a detection antibody, labelled, capable of binding the captured HCV antigen and secondly an antibody, anti-immunoglobulin or anti-isotype, or a detection antigen, labelled, capable of binding the captured anti-HCV antibody,
the antigen to capture, and the antigen to detect, an anti-HCV antibody comprising antigenic fragments of the capsid protein of the HCV, two epitopes of which have been destroyed,
and the capture and detection antibodies each recognizing one of said epitopes, intact, of the captured capsid antigen.

15. Method according to Claim 14, wherein the capture antibody and the detection antibody each recognize an epitope selected from the group of epitopes of the following sequences:
⁴⁴LGVR⁴⁷ (SEQ ID NO : 19), ³⁰IVGGVYL³⁶ (SEQ ID NO: 20) and ²⁹QIVGGV³⁴ (SEQ ID NO: 21).

16. Detection method according to Claim 6 or 7, comprising
a) bringing the sample together with a capture antibody of the HIV and a capture antigen of the HIV fixed on a solid phase;
b) incubating the mixture in conditions which allow the formation of antigen / antibody complexes;
c) separating the solid phase and the liquid phase;
d) bringing the solid phase into contact with a detection antibody, labelled, capable of binding the captured HIV antigen and an anti-immunoglobulin or anti-isotype antibody or antibodies, labelled, capable of binding the captured anti-HIV antibody,
the antigen to capture an anti-HIV antibody comprising an antigenic fragment of the gag protein of the HIV, at least one epitope of which has been destroyed,
and the capture and detection antibodies each recognizing one of said epitopes, intact, of the captured capsid antigen.

17. Method according to Claim 16, wherein the capture antibody and the detection antibody each recognize an epitope of the sequence QASQEVKNWMTETLL (SEQ ID NO: 24).

18. Method according to any one of Claims 14 to 17, wherein bringing the biological sample together with said capture antibody and said capture antigen fixed on a solid phase is done in the presence of at least one detergent of the non-ionic type.

19. Method according to Claim 18, wherein said non-ionic detergent is NP40.

20. Kit suitable for the detection of an infection by a hepatitis C virus (HCV) or a human immunodeficiency virus (HIV) in a biological sample, comprising
- a capture antigen immobilized on a solid phase, which comprises an antigenic fragment of a protein of said HCV or HIV, said fragment comprising at least one destroyed epitope, being made incapable of being recognized by a capture antibody and a detection antibody directed against said at least one epitope, intact, selected from the group of epitopes of the following sequences: ⁴⁴LGVR⁴⁷ (SEQ ID NO: 19), 30IVGGVYL³⁶ (SEQ ID NO: 20), ²⁹QIVGGV³⁴ (SEQ ID NO: 21), QASQEVKNWMTETLL (SEQ ID NO: 24) and QTDPAVKNWMTQTLL (SEQ ID NO: 25), whilst preserving the capacity to bind the antibodies directed against the micro-organism which are possibly present in the biological sample, by recognition of other epitopic sites which remain intact;
- said monospecific monoclonal or polyclonal detection antibody directed against said at least one epitope, intact, of said protein of the HCV or HIV, and
- said monospecific monoclonal or polyclonal capture antibody immobilized on a solid phase, directed against said at least one epitope, intact, of said protein of the HCV or HIV,
said protein of the HCV being the capsid protein of the HCV and the capture and/or detection antibody recognizing an epitope selected from the group of epitopes of the following sequences: ⁴⁴LGVR⁴⁷ (SEQ ID NO: 19), ³⁰IVGGVYL³⁶ (SEQ ID NO: 20) and ²⁹QIVGGV³⁴ (SEQ ID NO: 21), or
said protein of the HIV being the protein P25 of the HIV-1 and the capture and/or detection antibody recognizing the epitope of the sequence QASQEVKNWMTETLL (SEQ ID NO: 24), or
said protein of the HIV being the protein P26 of the HIV-2 and the capture and/or detection antibody recognizing the epitope of the sequence QTDPAVKNWMTQTLL (SEQ ID NO: 25).

21. Kit according to Claim 20, wherein said capture and detection antibodies are directed against an identical epitope, intact, of said protein of the HCV or HIV.

22. Kit according to Claim 20, wherein
- said capture antigen comprises an antigenic fragment of said protein of the HCV or HIV, said fragment comprising at least two destroyed epitopes being made incapable of being recognized by said capture antibody and said detection antibody, and
- said capture antibody is directed against one of said two epitopes, intact, of said protein of the HCV or HIV, whereas the detection antibody is directed against the other of said two epitopes, intact, of said protein of the HCV or HIV.

23. Kit according to one of Claims 20 to 22, comprising, in addition to the capture antigen, a means for detecting said anti-HCV antibodies present in the biological sample and complexed with said capture antigen.

24. Kit according to one of Claims 20 to 22, comprising, in addition to the capture antigen, a means for detecting said anti-HIV antibodies present in the biological sample and complexed with said capture antigen.

25. Kit according to Claim 23 or 24, wherein the detection means is a labelled anti-immunoglobulin or anti-isotype antibody.

26. Kit according to one of Claims 20 to 23, comprising
a) a capture antigen which comprises an antigenic fragment of a protein of the HCV, said fragment comprising at least two destroyed epitopes, whilst preserving the capacity to bind any anti-HCV antibodies present in a biological sample;
b) a capture antibody directed against one of said epitopes, intact, of said protein of the HCV, said capture antigen and said capture antibody being immobilized on a solid phase, and
c1) a detection antibody, labelled, directed against another of said epitopes, intact, of said protein of HCV, and/or
c2) optionally a detection antigen, labelled, which is an antigenic fragment of a protein of the HCV, said fragment comprising at least one destroyed epitope, whilst preserving the capacity to bind any anti-HCV antibodies present in a biological sample.

27. Kit according to Claim 25, comprising
a) the capture antigen which comprises an antigenic fragment of a protein of an HIV, said fragment comprising at least one destroyed epitope, whilst preserving the capacity to bind any anti-HIV antibodies present in a biological sample;
b) a capture antibody directed against one of said epitopes, intact, of said protein of the HIV, said capture antigen and said capture antibody being immobilized on a solid phase,
c1) a detection antibody, labelled, directed against another of said epitopes, intact, of said protein of the HIV,
c2) and/or optionally a detection antigen, labelled, which is an antigenic fragment of a protein of the HIV, said fragment comprising at least one destroyed epitope, whilst preserving the capacity to bind any anti-HIV antibodies present in a biological sample.

28. Kit according to one of Claims 20 to 27, further comprising, at least one detergent of the non-ionic type.

29. Kit according to Claim 28, wherein said detergent of the non-ionic type is NP40.

30. Peptide or polypeptide resulting from the capsid protein of the HCV, having at least one intact epitopic site and comprising any one of the sequences SEQ ID NO: 4 to SEQ ID NO: 18.

31. A peptide or polypeptide resulting from the gag protein of an HIV, having at least one intact epitopic site and comprising the sequence SEQ ID NO: 22.
